# EUROPEAN PATENT APPLICATION

(11) **EP 4 293 047 A1**
(43) Date of publication of application: **20.12.2023**
(21) Application number: 22752268.7
(22) Date of filing: 09.02.2022
(51) Int. Cl.: C07K 16/28, C12N 15/13, A61K 39/395, A61P 35/00

(54) **ANTI-PD-L1 ANTIBODY AND USE THEREOF**

(30) Priority: 10.02.2021 CN 202110183554
(71) Applicant: Shanghai Jemincare Pharmaceuticals Co., Ltd., Pudong New Area, Shanghai 201203 (CN); Jiangxi Jemincare Group Co., Ltd., Nanchang, Jiangxi 330096 (CN)
(72) Inventor: WANG, Zongda, Shanghai 201203 (CN); SONG, Jianqiu, Shanghai 201203 (CN); LIU, Xiaowu, Shanghai 201203 (CN); CAO, Xiaodan, Shanghai 201203 (CN); DENG, Sujun, Shanghai 201203 (CN); PAN, Zhongzong, Shanghai 201203 (CN); WANG, Xueping, Shanghai 201203 (CN)
(74) Representative: Bianchetti & Minoja with Trevisan & Cuonzo IPS SRL
(86) International application number: PCT/CN2022/075599
(87) International publication number: WO 2022/171108

(57) **Abstract**

The present application provides an isolated antigen-binding protein, which can bind to PD-L1 protein, block the binding of PD-1 protein with PD-L1 protein, and/or stimulate immune cells to secrete cytokines. The present application also provides the use of the isolated antigen-binding protein in the manufacture of a medicament.

## Description

### FIELD OF THE INVENTION

The present application relates to the field of biomedicine, in particular to an anti-PD-L1 antibody and its use in the manufacture of a medicament.

### BACKGROUND OF THE INVENTION

Malignant tumor is a disease that seriously threaten human health worldwide at present, and it is the main type of disease that causes human death. With the aging of population in China, the incidence of cancer is increasing, and effective therapeutic drugs need to be developed urgently, among which the development of immune checkpoint drugs has become a research hotspot in recent years.

The mechanism of PD-1 or PD-L1 immunotherapy is to design specific monoclonal antibodies against PD-1 or PD-L1, prevent the recognition of PD-1 and PD-L1, and restore the normal function of T cells, so that T cells can effectively kill tumor cells. Currently, many therapeutic antibodies have been developed against this signaling pathway, such as Durvalumab and Atezolizumab. However, during treating, there are widely existing phenomena such as low response rate and susceptible drug resistance. Therefore, an anti-tumor PD-L1 antibody with stable structure, excellent therapeutic effect and suitable for large-scale industrial production is urgently needed.

### SUMMARY OF THE INVENTION

The present application provides an isolated antigen-binding protein having one or more of the following properties: (1) capable of binding to PD-L1 protein derived from a primate with a *K_{D}* value of 1 × 10⁹ M or less; (2) capable of blocking the binding of PD-L1 protein to PD-1 protein; and (3) capable of stimulating the secretion of cytokines in immune cells.

In some embodiments, the primate includes a human and/or a monkey.

In some embodiments, the isolated antigen-binding protein includes an antibody or an antigen-binding fragment thereof.

In some embodiments, the antigen-binding fragment includes Fab, Fab', F(ab)₂, Fv fragment, F(ab')₂, scFv, di-scFv, VHH and/or dAb.

In some embodiments, the antibody is selected from the group consisting of monoclonal antibody, chimeric antibody, humanized antibody and fully human antibody.

In some embodiments, the isolated antigen-binding protein competes with a reference antibody for binding to PD-L1 protein, wherein the reference antibody comprises a heavy chain variable region VH and a light chain variable region VL, the VH of the reference antibody comprises HCDR1, HCDR2 and HCDR3, the VH of the reference antibody comprises HCDR1, HCDR2 and HCDR3, the VL of the reference antibody comprises LCDR1, LCDR2 and LCDR3, and the HCDR1 of the reference antibody comprises an amino acid sequence as set forth in SEQ ID NO: 1, the HCDR2 of the reference antibody comprises an amino acid sequence as set forth in SEQ ID NO: 2, the HCDR3 of the reference antibody comprises an amino acid sequence as set forth in SEQ ID NO: 3, the LCDR1 of the reference antibody comprises an amino acid sequence as set forth in SEQ ID NO: 4, the LCDR2 of the reference antibody comprises an amino acid sequence as set forth in SEQ ID NO: 5, and the LCDR3 of the reference antibody comprises an amino acid sequence as set forth in SEQ ID NO: 6.

In some embodiments, the isolated antigen-binding protein comprises HCDR3, and the HCDR3 comprises an amino acid sequence as set forth in SEQ ID NO: 3.

In some embodiments, the isolated antigen-binding protein comprises HCDR2, and the HCDR2 comprises an amino acid sequence as set forth in SEQ ID NO: 2.

In some embodiments, the isolated antigen-binding protein comprises HCDR1, and the HCDR1 comprises an amino acid sequence as set forth in SEQ ID NO: 1.

In some embodiments, the isolated antigen-binding protein comprises HCDR1, HCDR2 and HCDR3, wherein the HCDR1 comprises an amino acid sequence as set forth in SEQ ID NO: 1, the HCDR2 comprises an amino acid sequence as set forth in SEQ ID NO: 2, and the HCDR3 comprises an amino acid sequence as set forth in SEQ ID NO: 3.

In some embodiments, the isolated antigen-binding protein comprises a heavy chain variable region VH, wherein the VH comprises a framework region H-FR1, and the C-terminus of the H-FR1 is directly or indirectly linked to the N-terminus of the HCDR1, and the H-FR1 comprises an amino acid sequence as set forth in SEQ ID NO: 50.

In some embodiments, the H-FR1 comprises an amino acid sequence as set forth in any one of SEQ ID NO: 7 or 8.

In some embodiments, the VH comprises a framework region H-FR2, the H-FR2 is located between the HCDR1 and the HCDR2, and the H-FR2 comprises an amino acid sequence as set forth in SEQ ID NO: 51.

In some embodiments, the H-FR2 comprises an amino acid sequence as set forth in any one of SEQ ID NO: 9 to 11.

In some embodiments, the VH comprises a framework region H-FR3, the H-FR3 is located between the HCDR2 and the HCDR3, and the H-FR3 comprises an amino acid sequence as set forth in SEQ ID NO: 52.

In some embodiments, the H-FR3 comprises an amino acid sequence as set forth in any one of SEQ ID NO: 12 to 17.

In some embodiments, the VH comprises a framework region H-FR4, the N-terminus of the H-FR4 is linked to the C-terminus of the HCDR3, and the H-FR4 comprises an amino acid sequence as set forth in SEQ ID NO: 53.

In some embodiments, the H-FR4 comprises an amino acid sequence as set forth in SEQ ID NO: 18 or 19.

In some embodiments, the isolated antigen-binding protein comprises H-FR1, H-FR2, H-FR3 and H-FR4, wherein the H-FR1 comprises an amino acid sequence as set forth in SEQ ID NO: 7 or 8, the H-FR2 comprises an amino acid sequence as set forth in any one of SEQ ID NO: 9 to 11, the H-FR3 comprises an amino acid sequence as set forth in any one of SEQ ID NO: 12 to 17 and the H-FR4 comprises an amino acid sequence as set forth in SEQ ID NO: 18 or 19.

In some embodiments, the isolated antigen-binding protein comprises H-FR1, H-FR2, H-FR3 and H-FR4, and the H-FR1, H-FR2, H-FR3 and H-FR4 are selected from any one of groups of amino acid sequences as follows:
(1) H-FR1: SEQ ID NO: 7, H-FR2: SEQ ID NO: 9, H-FR3: SEQ ID NO: 12 and H-FR4: SEQ ID NO: 18;
(2) H-FR1: SEQ ID NO: 8, H-FR2: SEQ ID NO: 10, H-FR3: SEQ ID NO: 13 and H-FR4: SEQ ID NO: 19;
(3) H-FR1: SEQ ID NO: 8, H-FR2: SEQ ID NO: 11, H-FR3: SEQ ID NO: 14 and H-FR4: SEQ ID NO: 18;
(4) H-FR1: SEQ ID NO: 8, H-FR2: SEQ ID NO: 10, H-FR3: SEQ ID NO: 15 and H-FR4: SEQ ID NO: 19;
(5) H-FR1: SEQ ID NO: 8, H-FR2: SEQ ID NO: 10, H-FR3: SEQ ID NO: 16 and H-FR4: SEQ ID NO: 19; and
(6) H-FR1: SEQ ID NO: 8, H-FR2: SEQ ID NO: 11, H-FR3: SEQ ID NO: 17 and H-FR4: SEQ ID NO: 19.

In some embodiments, the isolated antigen-binding protein comprises a heavy chain variable region VH, and the VH comprises an amino acid sequence as set forth in SEQ ID NO: 54.

In some embodiments, the VH comprises an amino acid sequence as set forth in any one of SEQ ID NO: 20 to 25.

In some embodiments, the isolated antigen-binding protein comprises a heavy chain constant region, and the heavy chain constant region is derived from a human IgG constant region.

In some embodiments, the heavy chain constant region is derived from a human IgG1 constant region, and the human IgG1 constant region comprises an amino acid sequence as set forth in SEQ ID NO: 41.

In some embodiments, the isolated antigen-binding protein comprises an antibody heavy chain HC, and the HC comprises an amino acid sequence as set forth in any one of SEQ ID NO: 42 to 46.

In some embodiments, the isolated antigen-binding protein comprises LCDR3, and the LCDR3 comprises an amino acid sequence as set forth in SEQ ID NO: 6.

In some embodiments, the isolated antigen-binding protein comprises LCDR2, and the LCDR2 comprises an amino acid sequence as set forth in SEQ ID NO: 5.

In some embodiments, the isolated antigen-binding protein comprises LCDR1, and the LCDR1 comprises an amino acid sequence as set forth in SEQ ID NO: 4.

In some embodiments, the isolated antigen-binding protein comprises LCDR1, LCDR2 and LCDR3, wherein the LCDR1 comprises an amino acid sequence as set forth in SEQ ID NO: 4, the LCDR2 comprises an amino acid sequence as set forth in SEQ ID NO: 5, and the LCDR3 comprises an amino acid sequence as set forth in SEQ ID NO: 6.

In some embodiments, the isolated antigen-binding protein comprises a light chain variable region VL, wherein the VL comprises a framework region L-FR1, the C-terminus of the L-FR1 is directly or indirectly linked to the N-terminus of the LCDR1, and the L-FR1 comprises an amino acid sequence as set forth in SEQ ID NO: 55.

In some embodiments, the L-FR1 comprises an amino acid sequence as set forth in SEQ ID NO: 26 or 27.

In some embodiments, the VL comprises a framework region L-FR2, the L-FR2 is located between the LCDR1 and the LCDR2, and the L-FR2 comprises an amino acid sequence as set forth in SEQ ID NO: 56.

In some embodiments, the L-FR2 comprises an amino acid sequence as set forth in SEQ ID NO: 28 or 29.

In some embodiments, the VL comprises a framework region L-FR3, the L-FR3 is located between the LCDR2 and the LCDR3, and the L-FR3 comprises an amino acid sequence as set forth in SEQ ID NO: 57.

In some embodiments, the L-FR3 comprises an amino acid sequence as set forth in any one of SEQ ID NO: 30 to 33.

In some embodiments, the VL comprises a framework region L-FR4, the N-terminus of the L-FR4 is linked to the C-terminus of the LCDR3, and the L-FR4 comprises an amino acid sequence as set forth in SEQ ID NO: 58.

In some embodiments, the L-FR4 comprises an amino acid sequence as set forth in SEQ ID NO: 34 or 35.

In some embodiments, the isolated antigen-binding protein comprises L-FR1, L-FR2, L-FR3 and L-FR4, wherein the L-FR1 comprises an amino acid sequence as set forth in SEQ ID NO: 26 or 27, the L-FR2 comprises an amino acid sequence as set forth in SEQ ID NO: 28 or 29, the L-FR3 comprises an amino acid sequence as set forth in any one of SEQ ID NO: 30 to 33, and the L-FR4 comprises an amino acid sequence as set forth in SEQ ID NO: 34 or 35.

In some embodiments, the isolated antigen-binding protein comprises L-FR1, L-FR2, L-FR3 and L-FR4, and the L-FR1, L-FR2, L-FR3 and L-FR4 are selected from any one of groups of amino acid sequences as follows:
(1) L-FR1: SEQ ID NO: 26, L-FR2: SEQ ID NO: 28, L-FR3: SEQ ID NO: 30 and L-FR4: SEQ ID NO: 34;
(2) L-FR1: SEQ ID NO: 27, L-FR2: SEQ ID NO: 29, L-FR3: SEQ ID NO: 31 and L-FR4: SEQ ID NO: 35;
(3) L-FR1: SEQ ID NO: 27, L-FR2: SEQ ID NO: 29, L-FR3: SEQ ID NO: 32 and L-FR4: SEQ ID NO: 35; and
(4) L-FR1: SEQ ID NO: 27, L-FR2: SEQ ID NO: 29, L-FR3: SEQ ID NO: 33 and L-FR4: SEQ ID NO: 35.

In some embodiments, the isolated antigen-binding protein comprises a light chain variable region VL, and the VL comprises an amino acid sequence as set forth in SEQ ID NO: 59.

In some embodiments, the VL comprises an amino acid sequence as set forth in any one of SEQ ID NO: 36 to 39.

In some embodiments, the isolated antigen-binding protein comprises an antibody light chain constant region, and the antibody light chain constant region comprises an amino acid sequence as set forth in SEQ ID NO: 40.

In some embodiments, the isolated antigen-binding protein comprises an antibody light chain LC, and the LC comprises an amino acid sequence as set forth in any one of SEQ ID NO: 47 to 49.

In another aspect, the present application provides a polypeptide comprising the isolated antigen-binding protein.

In another aspect, the present application provides an immunoconjugate comprising the isolated antigen-binding protein or the polypeptide.

In another aspect, the present application provides an isolated nucleic acid molecule(s) encoding the isolated antigen-binding protein.

In another aspect, the present application provides a vector comprising the nucleic acid molecule.

In another aspect, the present application provides a cell comprising the nucleic acid molecule or the vector.

In another aspect, the present application provides a method for preparing the isolated antigen-binding protein, comprising culturing the cell under conditions that the isolated antigen-binding protein can be expressed.

In another aspect, the present application provides a pharmaceutical composition comprising the isolated antigen-binding protein, the nucleic acid molecule, the vector and/or the cell, and optionally a pharmaceutically acceptable carrier.

In another aspect, the present application provides the use of the isolated antigen-binding protein, the nucleic acid molecule, the vector, the cell and/or the pharmaceutical composition in the manufacture of a medicament for preventing, alleviating and/or treating a tumor.

In another aspect, the present application provides a method for preventing, alleviating or treating a tumor, comprising administering the isolated antigen-binding protein and/or the polypeptide to a subject in need.

In another aspect, the present application provides the isolated antigen-binding protein, for use in preventing, alleviating or treating a tumor.

In some embodiments, the tumor comprises a tumor with a high expression of PD-1 or PD-L1.

In some embodiments, the tumor comprises a solid tumor.

In some embodiments, the tumor comprises breast cancer, lung cancer, gastric cancer and urothelial carcinoma.

In another aspect, the present application provides a method for inhibiting the binding of PD-1 protein to PD-L1 protein, comprising administering the isolated antigen-binding protein and/or the polypeptide.

In another aspect, the present application provides a method for stimulating the secretion of cytokines in immune cells, comprising administering the isolated antigen-binding protein and/or the polypeptide.

In another aspect, the present application provides a method for detecting the presence and/or content of PD-L1 protein, comprising administering the isolated antigen-binding protein and/or the polypeptide.

In another aspect, the present application provides a kit comprising the isolated antigen-binding protein, the polypeptide, the immunoconjugate, the nucleic acid molecule, the vector, the cell and/or the pharmaceutical composition.

Other aspects and advantages of the present invention will be readily apparent to those skilled in the art from the following detailed description. In the following detailed description, only exemplary embodiments of the present invention are shown and described. As will be recognized by those skilled in the art, the contents of the present application enable those skilled in the art to modify the disclosed specific embodiments without departing from the spirit and scope of the present application to which the present application relates. Accordingly, the descriptions in the drawings and specification of the present application are only exemplary but not restrictive.

### BRIEF DESCRIPTION OF DRAWINGS

The specific features of the present application to which the present application relates are shown in the appended claims. The features and advantages of the present application to which the present application relates will be better understood by referring to the exemplary embodiments and drawings described in detail below. A brief description of the attached drawings is as follows:
Fig. 1 shows that the antigen-binding protein described in the present application binds to human PD-L1 protein on HEK293 cell.
Fig. 2 shows that the antigen-binding protein described in the present application binds to monkey PD-L1 protein on CHOK1 cell.
Fig. 3 shows that the antigen-binding protein described in the present application blocks the binding of human PD-1 to human PD-L1 protein on HEK293 cell.
Fig. 4 shows that the antigen-binding protein described in the present application blocks the binding of human PD-1 to monkey PD-L1 protein on CHOK1 cell.
Fig. 5 shows that the antigen-binding protein described in the present application stimulates immune cells to secrete cytokine IL-2.

### DETAILED DESCRIPTION OF THE INVENTION

The embodiments of the present application will be described in the following by specific examples, and those skilled in the art can easily understand other advantages and effects of the present application from the disclosure in this specification.

### Definition of Terms

In the present application, the term "PD-1" generally refers to the programmed death 1 receptor, which is also be called "programmed death 1", "CD279", "differentiation cluster 279", "PD1", "PDCD1" or "CD297". PD-1 protein usually comprises an extracellular IgV domain, a transmembrane domain and an intracellular tail. PD-1 is usually expressed on T cells, B cells, natural killer T cells, activated monocytes and dendritic cells (DC). PD-1 can bind to its ligands PD-L1 and PD-L2. The term "PD-1" encompasses any natural PD-1 or modified PD-1 derived from any vertebrates, including mammals, such as primates (for example, human or monkeys) and rodents (for example, mice or rats). The term encompasses "full-length", unprocessed PD-1 and any form of PD-1 produced by processing in cells. PD-1 can exist as a transmembrane protein or as a soluble protein. "PD-1" comprises complete PD-1 and fragments thereof, as well as functional variants, isoforms, homologues, derivatives and analogues of PD-1, and analogues with at least one common epitope with PD-1. PD-1 sequences are known in the art. For example, an exemplary full-length human PD-1 protein sequence can be found by NCBI Accession Number NP_005009.2, and an exemplary full-length cynomolgus monkey PD-1 protein sequence can be found by NCBI Accession Number NP_001271065 or Uniprot Accession Number B0LAJ3.

In the present application, the term "PD-L1" generally refers to the programmed death ligand 1 protein. PD-L1 is also called differentiation cluster 274 (CD274) or B7 homologue 1 (B7-H1), and it is a protein encoded by (human) CD274 gene. PD-L1 can bind to its receptor, such as programmed death 1 (PD-1). The complexation of PD-L1 and PD-1 plays an immunosuppressive role by inhibiting T cell proliferation and producing cytokines IL-2 and IFN-γ. The term "PD-L1" encompasses any natural PD-L1 or modified PD-1 derived from any vertebrates, including mammals, such as primates (for example, human or monkeys) and rodents (for example, mice or rats). The term encompasses "full-length", unprocessed PD-L1 and any form of PD-L1 produced by processing in cells. PD-L1 can exist as a transmembrane protein or as a soluble protein. The term also encompasses naturally occurring variants of PD-L1, such as splicing variants or allelic variants. The basic structure of PD-L1 comprises four domains: extracellular Ig-like V domain and Ig-like C2 domain, transmembrane domain and cytoplasmic domain. PD-L1 sequences are known in the art. For example, information about human PD-L1 gene (including genomic DNA sequence) can be found by NCBI Gene ID No.29126. The amino acid sequence of an exemplary full-length human PD-L1 protein can be found by NCBI Accession Number NP_054862 or UniProt Accession Number Q9NZQ7.

In the present application, the term "antigen-binding protein" generally refers to a protein containing a portion that binds to an antigen, and optionally a scaffold or skeleton part portion that allows the portion that binds to an antigen with a conformation that promotes the binding of the antigen-binding protein to an antigen. An antigen-binding protein may typically comprise an antibody light chain variable region (VL), an antibody heavy chain variable region (VH) or both of them, and functional fragments thereof. The variable regions of heavy and light chains contain binding domains that interact with antigens. Examples of antigen-binding proteins include but not limited to antibodies, antigen-binding fragments, immunoconjugates, multispecific antibodies (e.g., bispecific antibodies), antibody fragments, antibody derivatives, antibody analogs or fusion proteins, or the like, as long as they show the required antigen-binding activity.

In the present application, the term "antibody" generally refers to an immunoglobulin that is reactive to a specified protein or peptide or fragment thereof. Antibodies can be antibodies from any class, including but not limited to IgG, IgA, IgM, IgD and IgE, and antibodies from any subclass (e.g. IgG1, IgG2, IgG3, and IgG4). The antibody may comprise a heavy chain constant region selected from IgG1, IgG2, IgG3, or IgG4, for example. The antibody may also comprise a light chain selected from kappa (κ) or lambda (λ), for example. The antibodies of the present application can be derived from any species.

In the present application, the term "antigen-binding fragment" generally refers to a portion of an antibody molecule, which contains an amino acid residue that interacts with an antigen and gives the antibody specificity and affinity to an antigen. Examples of antigen-binding fragments may include but not limited to Fab, Fab', F(ab)2, a Fv fragment, F(ab')₂, scFv, di-scFv and/or dAb. In the present application, the term "Fab" generally refers to a fragment containing a heavy chain variable domain and a light chain variable domain, and also contains a constant domain of the light chain and a first constant domain (CH1) of the heavy chain; the term "Fab'" generally refers to a fragment different from Fab by adding a small amount of residues (including one or more cysteine from the antibody hinge region) to the carboxyl end of the CH1 domain of the heavy chain; the term "F(ab')₂" generally refers to the dimer of Fab', which contains antibody fragments of two Fab fragments connected by a disulfide bridge on the hinge region. The term "Fv" generally refers to a smallest antibody fragment containing complete antigen recognition and binding sites. In some embodiments, the fragment can be composed of a dimer with a heavy chain variable region and a light chain variable region in closely non-covalent bonding; the term "dsFv" generally refers to disulfide-stabilized Fv fragments, in which the bond between a single light chain variable region and a single heavy chain variable region is disulfide bond. The term "dAb fragment" generally refers to an antibody fragment composed of a VH domain. In the present application, the term "scFv" generally refers to a monovalent molecule formed by covalent connection and pairing of a heavy chain variable domain and a light chain variable domain of an antibody through a flexible peptide linker; such scFv molecule can comprise the general structure: NH₂-VL- linker-VH-COOH or NH₂-VH- linker-VL-COOH.

In the present application, the term "variable region" or "variable domain" generally refers to the domain of antibody heavy chain or light chain involved in the binding of antibody to antigen. In the present application, the term "variable" generally means that some portions of the sequence of the variable domain of an antibody change strongly, forming the binding and specificity of various specific antibodies to their specific antigens. Variability is distributed in the whole variable region of antibody unevenly. It mainly exists in three segments in the light chain variable region and the heavy chain variable region, which are called complementarity determining region (CDR) or high variable region (HVR), namely LCDR1, LCDR2, LCDR3, HCDR1, HCDR2 and HCDR3 respectively. The more highly conservative portion of the variable domain is called a framework region (FR). The variable domains of natural heavy chain and light chain comprise four FR regions (H-FR1, H-FR2, H-FR3, H-FR4, L-FR1, L-FR2, L-FR3, L-FR4) respectively, and most of them have β-folding configuration and are connected by three CDR structural loop regions. CDRs in each chain are close together through FR regions, and together with the CDRs from the other chain form an antigen binding site of the antibody.

In this field, variable regions of antibodies or CDRs of antibodies can be numbered by various methods, such as Kabat numbering scheme and definition rules based on sequence variability (see Kabat et al., protein sequence in immunology, 5th Edition, National Institutes of Health, Bethesda, Maryland (1991)), Chothia numbering scheme and definition rules based on the position of structural loop regions (see, A1- Lazikani et al., J Mol Biol 273:927-48,1997), IMGT numbering scheme and definition rules based on amino acid sequence alignment of germline V gene by efranc *et al.,* and Honneger's numbering scheme (AHo's), Martin numbering scheme, Gelfand numbering scheme, etc. See Mathieu Dondelinger et al., Understanding the Significance and Implications of Antibody Numbering and Antigen-Binding Surface/Residue Definition, Front. Immunol., 16 October 2018.

In the present application, the term "monoclonal antibody" generally refers to an antibody obtained from a group of basically homogeneous antibodies, that is, each antibody constituting the group is the same, except for possible naturally occurring mutations and/or post-translational modifications (e.g., isomerization, amidation) that may exist in a very small amount. Monoclonal antibodies are highly specific and for a single antigenic site.

In the present application, the term "chimeric antibody" generally refers to an antibody in which the variable region is derived from one species and the constant region is derived from another species. Generally, the variable region is derived from an antibody of an experimental animal such as a rodent ("parent antibody"), and the constant region is derived from a human antibody, so that the obtained chimeric antibody is less likely to cause an adverse immune response in a human individual, compared with a parent (e.g., mouse-derived) antibody.

In the present application, the term "humanized antibody" generally refers to an antibody in which some or all of the amino acids other than the CDR region of a non-human antibody (e.g. a mouse antibody) are replaced by corresponding amino acids derived from human immunoglobulin. Addition, deletion, insertion, substitution or modification of amino acids in the CDR region can also be allowed, as long as they still retain the ability of antibodies to bind to specific antigens. A humanized antibody may optionally comprise at least a portion of the constant region of human immunoglobulin. A humanized antibody retains the antigen specificity similar to that of the original antibody. A "humanized" form of a non-human (e.g., murine) antibody may comprise a chimeric antibody derived from a sequence of a non-human immunoglobulin at a minimum. In some cases, the residues of CDR region in human immunoglobulin (receptor antibody) can be replaced by the residues of CDR region of a non-human species (donor antibody) (such as mice, rats, rabbits or non-human primates) with desired properties, affinity and/or ability. In some cases, the residues in the FR region of human immunoglobulin can be replaced by corresponding non-human residues. In addition, the humanized antibody may comprise amino acid modifications that are not present in the recipient antibody or in the donor antibody.

In the present application, the term "fully human antibody" generally refers to an antibody in which all portions (including the variable region and the constant region of the antibody) are encoded by genes of human origin. The methods to obtain fully human antibodies in this field may comprise phage display technology, transgenic mouse technology, ribosome display technology and RNA-polypeptide technology.

In the present application, the terms "binding", "specifically binding" or "specific for" generally refer to measurable and reproducible interactions, such as the binding of antigens with antibodies, which can determine the presence of a target in the presence of heterogeneous populations of molecules, including biological molecules. For example, an antibody binds to an epitope through its antigen-binding domain, and this binding requires some complementarity between the antigen-binding domain and the epitope. For example, an antibody that specifically binds to a target (which may be an epitope) is an antibody that binds to this target with greater affinity, binding affinity, and easier and/or longer duration than binding of the antibody to other targets. An antibody is called to "specifically bind" to an antigen when it is more likely to bind to an epitope through its antigen-binding domain than binding of the antibody to a random, unrelated epitope.

In the present application, the terms "KD" and *"K_{D}"* can be used interchangeably and usually refer to the equilibrium dissociation constant, and "KD" is a ratio of the dissociation rate constant (k_{dis}, also known as "off-rate (k_{off})" or "kd") to the association rate constant (kₒₙ, also known as "bonding rate (kₒₙ)" or "ka"). Association rate constant (kₒₙ), dissociation rate constant (k_{dis}) and equilibrium dissociation constant (KD) can be used to express the binding affinity of antigen-binding proteins (e.g. antibodies) to antigens. Methods for determining the association and dissociation rate constants are well known in the art, including but not limited to Biolayer interferometry (BLI), Radioimmunoassay (RIA), Equilibrium Dialysis, Surface Plasmon Resonance (SPR), Fluorescence Resonance Energy Transfer (FRET), co- immunoprecipitation (Co-IP) and protein Chip Technology. If measured under different conditions (e.g. salt concentration and pH), the measured affinity of a specific protein-protein interaction may be different.

In the present application, the term "primate" usually refers to monkey and ape species, and includes monkey species, such as the monkeys from the *Macaca* (such as, cynomolgus *(Macaca fascicularis*) and/or rhesus monkey *(Macaca mulatta*)) and baboon *(Papio ursinus),* as well as marmoset (a species from *Callithrix*)*,* squirrel monkey (a species from Saimiri), and tamarin (a species from *Saguinus),* and ape species, such as chimpanzee *(Pan troglodytes),* and also includes *Homo sapiens.*

In the present application, the terms "polypeptide" or "protein" are used interchangeably and generally refer to polymers of amino acid residues. The term also applies to amino acid polymers in which one or more amino acid residues are analogs or mimetics of corresponding naturally occurring amino acids, as well as naturally occurring amino acid polymers. The term may also comprise modified amino acid polymers, for example, by adding sugar residues to form glycoproteins or modification of phosphorylation. Polypeptides and proteins can be produced by naturally occurring and non-recombinant cells or genetically engineered or recombinant cells, and may comprise molecules with amino acid sequences of natural protein, or molecules with deletion, addition and/or substitution of one or more amino acids of natural sequences. The terms "polypeptide" and "protein" particularly comprise deletion, addition and/or substitution of one or more amino acids of a sequence of the antigen-binding protein described in the present application.

In the present application, the term "homologue" generally refers to an amino acid sequence or nucleotide sequence that has certain homology with the wild-type amino acid sequence and the wild-type nucleotide sequence. The term "homology" can be equivalent to sequence "identity". Homologous sequences may comprise amino acid sequences that may be at least 80%, 85%, 90%, 99.1%, 99.2%, 99.3%, 99.4%, 99.5%, 99.6%, 99.7%, 99.8% or 99.9% identical to the subject sequence. Typically, the homologue will comprise the same active site or the like as the subject amino acid sequence. Homology may be considered in terms of similarity (i.e., amino acid residues having similar chemical properties/functions), or may be expressed in terms of sequence identity. In the present application, a sequence having percentage identity in any one of the mentioned SEQ ID NO of a mentioned amino acid sequence or nucleotide sequence refers to a sequence having the percentage identity over the entire length of the mentioned SEQ ID NO.

In the present application, the term "isolated" generally refers to biological materials (e.g. viruses, nucleic acids, or protein) that are substantially free of components normally associated with or interacting with their naturally occurring environment. The isolated biological materials optionally includes additional materials that are not found in its natural environment (e.g., nucleic acids or proteins). In the present application, when it comes to protein, "isolation" usually means that the molecule is isolated and separated from the whole organism in which the molecule is found to exist naturally, or there are basically no other biomacromolecules of the same type. When a nucleic acid molecule is referred to, it is completely or partially separated from the sequence that naturally binds to it, or the nucleic acid has a heterologous sequence that binds to it, or the nucleic acid is separated from the chromosome.

In the present application, the term "immunoconjugate" generally refers to the substance formed by the connection of antigen-binding protein with other active agents, which may be a small molecular active agent, such as a chemotherapeutic agent, a toxin, an immunotherapy agent, an imaging probe or a fluorescent probe.

In the present application, the term "nucleic acid" molecule generally refers to any length of isolated form of nucleotide, deoxynucleotide or ribonucleotide or its analogues isolated from its natural environment or artificially synthesized.

In the present application, the term "vector" generally refers to a nucleic acid molecule capable of self-replication in a suitable host, which transfers the inserted nucleic acid molecule into and/or between host cells. The vector may comprise a vector mainly used for inserting DNA or RNA into cells, a vector mainly used for replicating DNA or RNA, and a vector mainly used for transcription and/or expression of translation of DNA or RNA. The vector also comprises a vector with a plurality of the above functions. The vector can be a polynucleotide that can be transcribed and translated into a polypeptide when introduced into a suitable host cell. Generally, the vector can produce a desired expression product by culturing a suitable host cell comprising the vector.

In the present application, the term "cell" generally refers to an individual cell, cell line or cell culture that may comprise or already has comprised a plasmid or vector including a nucleic acid molecule described in the present application, or that can express an antigen-binding protein described in the present application. The cells may comprise progeny of a single host cell. Because of natural, unexpected or intentional mutation, the progeny cells may not be exactly the same as the original parent cells in morphology or genome, but they can express the antibody or antigen-binding fragment thereof described in the present application. The cells can be obtained by transfecting cells in vitro using the vector described in the present application. The cells can be prokaryotic cells (e.g. *Escherichia coli*) or eukaryotic cells (e.g. yeast cells, e.g. COS cells, China hamster ovary (CHO) cells, HeLa cells, HEK293 cells, COS-1 cells, NS0 cells or myeloma cells). In some cases, the cells may be mammalian cells. For example, the mammalian cells may be CHO-K1 cells.

In the present application, the term "pharmaceutical composition" generally refers to a preparation that exists in a form that allows the biological activity of an active ingredient to be effective, and does not contain additional ingredients with unacceptable toxicity to a subject to whom the composition is to be administered.

In the present application, the term "treatment" generally refers to the desire to change the natural course of the individual being treated, and can be clinical intervention to achieve prevention and treatment or in the process of clinical lesions. Desirable therapeutic effects comprise, but not limited to, preventing the occurrence or recurrence of the disease, relieving symptoms, weakening any direct or indirect pathological consequences of the disease, preventing metastasis, reducing the rate of disease progression, improving or alleviating the disease state and alleviating or improving the prognosis. In some cases, the antigen-binding protein (e.g. anti-PD-L1 antibody) of the present application can be used to delay disease development or slow disease progression.

In the present application, the term "administration" generally refers to a method of administering a certain dose of a compound (e.g., an anticancer therapeutic agent) or a pharmaceutical composition (e.g., a pharmaceutical composition comprising an anticancer therapeutic agent) to a subject (e.g., a patient). Administration can be carried out by any suitable means, including parenteral, intrapulmonary and intranasal administration, and (if necessary for local treatment) intralesional administration. Parenteral infusion comprises, for example, intramuscular, intravenous, intra-arterial, intraperitoneal or subcutaneous administration.

In the present application, the term "tumor" generally refers to the growth and proliferation of all neoplastic cell (either malignant or benign) and all precancerous and cancerous cells and tissues. In the present application, the tumor can be a tumor with a high expression of PD-1 or PD-L1 in cells and tissues. Tumors may comprise solid tumors and/or non-solid tumors (e.g., hematomas, lymphomas).

In the present application, the term "between" generally means that the C-terminus of an amino acid fragment is directly or indirectly linked to the N-terminus of a first amino acid fragment, and its N-terminus is directly or indirectly linked to the C-terminus of a second amino acid fragment. In the light chain, for example, the N-terminus of the L-FR2 is directly or indirectly linked to the C-terminus of the LCDR1, and the C-terminus of the L-FR2 is directly or indirectly linked to the N-terminus of the LCDR2. For another example, the N-terminus of the L-FR3 is directly or indirectly linked to the C-terminus of the LCDR2, and the C-terminus of the L-FR3 is directly or indirectly linked to the N-terminus of the LCDR3. In the heavy chain, for example, the N-terminus of the H-FR2 is directly or indirectly linked to the C-terminus of the HCDR1, and the C-terminus of the H-FR2 is directly or indirectly linked to the N-terminus of the HCDR2. For another example, the N-terminus of the H-FR3 is directly or indirectly linked to the C-terminus of the HCDR2, and the C-terminus of the H-FR3 is directly or indirectly linked to the N-terminus of the HCDR3. In the present application, the "first amino acid fragment" and the "second amino acid fragment" can be any one of the same or different amino acid fragments.

In the present application, the term "includes/include" generally refers to the meaning of comprising, inclusive, containing, or encompassing. In some cases, it also represents the meanings of "is", and "consist of'.

In the present application, the term "about" generally refers to a change in the range of 0.5%-10% above or below a specified value, such as a change in the range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below a specified value.

### Detailed Description of the Present Application

### Antigen Binding Protein

In an aspect, the present application provides an isolated antigen-binding protein, which can bind to PD-L1 derived from primates (e.g. humans or monkeys) with a KD value of 1 × 10⁻⁹ M or less. The binding affinity of primate PD-L1 antigen-binding protein to PD-L1 can be determined by any method known in the art. In some cases, the binding affinity can be determined by surface plasmon resonance (SPR), enzyme-linked immunosorbent assay (ELISA), binding antigen precipitation method, equilibrium dialysis method and biolayer interference (BLI). In some cases, the binding affinity and KD value of PD-L1 antigen-binding protein to PD-L1 can be determined by biolayer interference (BLI). For example, an ForteBio Octet molecular interaction analyzer can be used to analyze the binding kinetics between antigens and antibodies.

In the present application, the isolated antigen-binding protein can bind to PD-L1 derived from primates with a KD value of 1 × 10⁻⁹ M or less. For example, the KD value can be a value of about 1 × 10 ⁻⁹ M or less, about 9 × 10⁻¹⁰ M or less, about 8 × 10⁻¹⁰ M or less, about 7 × 10⁻¹⁰ M or less, about 6 × 10⁻¹⁰ M or less, about 5 × 10⁻¹⁰ M or less, about 4 × 10⁻¹⁰ M or less, about 3 × 10⁻¹⁰ M or less, about 2 × 10⁻¹⁰ M or less, and about 1 × 10⁻¹⁰ M or less binding to PD-L1 derived from humans, for example, detected using a FortieBio Octet molecular interaction analyzer.

In another case, the binding activity of PD-L1 antigen-binding protein described in the present application to PD-L1 can be detected by flow cytometry or enzyme-linked immunosorbent assay.

For example, in FACS test, host cells stably expressing human PD-L1 (such as HEK293 cells) are used, and the EC50 value of the PD-L1 antigen-binding protein binding to PD-L1 is between about 0.0001 µg/mL and about 100 µg/mL, for example, between about 0.001 µg/mL and about 10 µg/mL, between about 0.001 µg/mL and about 5 µg/mL, between about 0.001 µg/mL and about 1 µg/mL, between about 0.01 µg/mL and about 0.5 µg/mL, between about 0.01 µg/mL and about 0.1 µg/mL, or between about 0.01 µg/mL and about 0.05 µg/mL.

For example, in FACS test, host cells stably expressing monkey PD-L1 (such as CHOK1 cells) are used, and the EC50 value of PD-L1 antigen-binding protein binding to PD-L1 is between about 0.0001 µg/mL and about 100 µg/mL, for example, between about 0.001 µg/mL and about 10 µg/mL, between about 0.001 µg/mL and about 5 µg/mLM, between about 0.01 µg/mL and about 1 µg/mL, between about 0.02 µg/mL and about 0.5 µg/mL, and between about 0.03 µg/mL and about 0.1 µg/mL.

In another aspect, the antigen-binding protein described in the present application can block the binding of PD-1 to PD-L1. In some cases, the blocking of the binding of PD-1 to PD-L1 by the antigen-binding protein can be determined by flow cytometer FACS or enzyme-linked immunosorbent assay (ELISA).

For example, host cells stably expressing human PD-L1 (such as HEK293 cells) are first incubated with a decreasing amount of unlabeled antigen-binding protein, and then incubated with biotin-labeled PD-1 protein. Then, cells were analyzed using FACS to confirm that the antigen-binding protein blocks the binding of PD-1 to PD-L1. For example, between about 0.001 µg/mL and about 10 µg/mL, between about 0.001 µg/mL and about 5 µg/mL, between about 0.01 µg/mL and about 1 µg/mL, between about 0.02 µg/mL and about 0.5 µg/mL, and between about 0.02 µg/mL and about 0.1 µg/mL.

For example, host cells stably expressing monkey PD-L1 (such as CHOK1 cells) are first incubated with a decreasing amount of unlabeled antigen-binding protein, and then incubated with biotin-labeled PD-1 protein. Then, cells were analyzed using FACS to confirm that the antigen-binding protein blocks the binding of PD-1 to PD-L1. For example, between about 0.001 µg/mL to about 10 µg/mL, about 0.001 µg/mL to about 5 µg/mL, about 0.01 µg/mL to about 1 µg/mL, and about 0.1 µg/mL to about 0.7 µg/mL.

In the present application, the isolated antigen-binding protein may comprise CDR3 from a heavy chain variable region VH, and the VH may comprise an amino acid sequence as set forth in SEQ ID NO: 54.

In the present application, the isolated antigen-binding protein may comprise HCDR3, and the HCDR3 may comprise CDR3 of VH with an amino acid sequence as set forth in any one of SEQ ID NO: 20 to 25. For example, the isolated antigen-binding protein may comprise HCDR3, and the HCDR3 may comprise an amino acid sequence as set forth in SEQ ID NO: 3.

In the present application, the isolated antigen-binding protein may comprise CDR2 from a heavy chain variable region VH, and the VH may comprise an amino acid sequence as set forth in SEQ ID NO: 54.

In the present application, the isolated antigen-binding protein may comprise HCDR2, and the HCDR2 may comprise CDR2 of VH with an amino acid sequence as set forth in any one of SEQ ID NO: 20 to 25. For example, the isolated antigen-binding protein may comprise HCDR2, and the HCDR2 may comprise an amino acid sequence as set forth in SEQ ID NO: 2.

In the present application, the isolated antigen-binding protein may comprise CDR1 from a heavy chain variable region VH, and the VH may comprise an amino acid sequence as set forth in SEQ ID NO: 54.

In the present application, the isolated antigen-binding protein may comprise HCDR1, and the HCDR1 may comprise CDR1 of VH with an amino acid sequence as set forth in any one of SEQ ID NO: 20 to 25. For example, the isolated antigen-binding protein may comprise HCDR1, and the HCDR1 may comprise an amino acid sequence as set forth in SEQ ID NO: 1.

In the present application, the isolated antigen-binding protein may comprise HCDR1, HCDR2 and HCDR3, the HCDR1 may comprise CDR1 of VH with an amino acid sequence as set forth in SEQ ID NO: 54, the HCDR2 may comprise CDR2 of VH with an amino acid sequence as set forth in SEQ ID NO: 54, and the HCDR3 may comprise CDR3 of VH with an amino acid sequence as set forth in SEQ ID NO: 54.

In the present application, the isolated antigen-binding protein may comprise HCDR1, HCDR2 and HCDR3, the HCDR1 may comprise CDR1 of VH with an amino acid sequence as set forth in any one of SEQ ID NO: 20 to 25, the HCDR2 may comprise CDR2 of VH with an amino acid sequence as set forth in any one of SEQ ID NO: 20 to 25, and the HCDR3 may comprise CDR3 of VH with an amino acid sequence as set forth in any one of SEQ ID NO: 20 to 25.

For example, the isolated antigen-binding protein may comprise CDR1 of VH with an amino acid sequence as set forth in SEQ ID NO: 20, may comprise CDR2 of VH with an amino acid sequence as set forth in SEQ ID NO: 20, and may comprise CDR3 of VH with an amino acid sequence as set forth in SEQ ID NO: 20.

For example, the isolated antigen-binding protein may comprise CDR1 of VH with an amino acid sequence as set forth in SEQ ID NO: 21, may comprise CDR2 of VH with an amino acid sequence as set forth in SEQ ID NO: 21, and may comprise CDR3 of VH with an amino acid sequence as set forth in SEQ ID NO: 21.

For example, the isolated antigen-binding protein may comprise CDR1 of VH with an amino acid sequence as set forth in SEQ ID NO: 22, may comprise CDR2 of VH with an amino acid sequence as set forth in SEQ ID NO: 22, and may comprise CDR3 of VH with an amino acid sequence as set forth in SEQ ID NO: 22.

For example, the isolated antigen-binding protein may comprise CDR1 of VH with an amino acid sequence as set forth in SEQ ID NO: 23, may comprise CDR2 of VH with an amino acid sequence as set forth in SEQ ID NO: 23, and may comprise CDR3 of VH with an amino acid sequence as set forth in SEQ ID NO: 23.

For example, the isolated antigen-binding protein may comprise CDR1 of VH with an amino acid sequence as set forth in SEQ ID NO: 24, may comprise CDR2 of VH with an amino acid sequence as set forth in SEQ ID NO: 24, and may comprise CDR3 of VH with an amino acid sequence as set forth in SEQ ID NO: 24.

For example, the isolated antigen-binding protein may comprise CDR1 of VH with an amino acid sequence as set forth in SEQ ID NO: 25, may comprise CDR2 of VH with an amino acid sequence as set forth in SEQ ID NO: 25, and may comprise CDR3 of VH with an amino acid sequence as set forth in SEQ ID NO: 25.

For example, the isolated antigen-binding protein described in the present application may comprise HCDR1, HCDR2 and HCDR3, the HCDR1 may comprise an amino acid sequence as set forth in SEQ ID NO: 1, the HCDR2 may comprise an amino acid sequence as set forth in SEQ ID NO: 2, and the HCDR3 may comprise an amino acid sequence as set forth in SEQ ID NO: 3.

In the present application, the isolated antigen-binding protein may comprise H-FR1, and the H-FR1 may comprise an amino acid sequence as set forth in SEQ ID NO: 50: EVQLVESGGGLVQPGX₁₆SLX₁₉LSCX₂₃AS (SEQ ID NO: 50), where X₁₆ is G or R, X₁₉ is K or R, and X₂₃ is A or V. For example, it may be divided according to Chothia rules.

In some cases, compared with an amino acid sequence as set forth in SEQ ID NO: 7, the H-FR1 may comprise at least amino acid substitutions at positions selected from the group consisting of amino acid substitutions at X₁₆, X₁₉ and/or X₂₃.

For example, the H-FR1 may comprise an amino acid sequence as set forth in SEQ ID NO: 7 or 8.

In the present application, the isolated antigen-binding protein may comprise H-FR2, and the H-FR2 may comprise an amino acid sequence as set forth in SEQ ID NO: 51: WMX₃WX₅RQAPGKGLEWVASI (SEQ ID NO: 51), where X₃ is S or T, and X₅ is I or V For example, it may be divided according to Chothia rules.

In some cases, compared with an amino acid sequence as set forth in SEQ ID NO: 9, the H-FR2 may comprise at least amino acid substitutions at positions selected from the group consisting of amino acid substitutions at X₃ and/or X₅.

For example, the H-FR2 may comprise an amino acid sequence as set forth in any one of SEQ ID NO: 9 to 11.

In the present application, the isolated antigen-binding protein may comprise H-FR3, and the H-FR3 may comprise an amino acid sequence as set forth in SEQ ID NO: 52: TYYX₄DSVKGRFTISRDX₁₇X₁₈KX₂₀X₂₁LYLQMNSLRX₃₁EDTAX₃₆YYCSR (SEQ ID NO: 52), where X₄ is A, P or V, X₁₇ is D or N, X₁₈ is A or S, X₂₀ is N or S, X₂₁ is S or T, X₃₁ is A or S, and X₃₆ is T or V For example, it may be divided according to Chothia rules.

In some cases, compared with an amino acid sequence as set forth in SEQ ID NO: 12, the H-FR3 may comprise at least amino acid substitutions at positions selected from the group consisting of amino acid substitutions at X₄, X₁₇, X₁₈, X₂₀, X₂₁, X₃₁ and/or X₃₆.

For example, the H-FR3 may comprise an amino acid sequence as set forth in any one of SEQ ID NO: 12 to 17.

In the present application, the isolated antigen-binding protein may comprise H-FR4, and the H-FR4 may comprise an amino acid sequence as set forth in SEQ ID NO: 53: WGQGX₅X₆VTVSS (SEQ ID NO: 53), where X₅ is T or V, and X₆ is L or M. For example, it may be divided according to Chothia rules.

In some cases, compared with an amino acid sequence as set forth in SEQ ID NO: 18, the H-FR4 may comprise at least amino acid substitution at X₆ and/or X₅.

For example, the H-FR4 may comprise an amino acid sequence as set forth in SEQ ID NO: 18 or 19.

In the present application, the isolated antigen-binding protein may comprise a heavy chain variable region VH, and the VH may comprise an amino acid sequence as set forth in SEQ ID NO: 54:
EVQLVESGGGLVQPGX₁₆SLX₁₉LSCX₂₃ASGFTFSNFWMX₃₅WX₃₇RQAPGKGLEWVASITHSG GITYYX₆₁DSVKGRFTISRD₇₄X₇₅KX₇₇X₇₈LYLQMNSLRX₈₈EDTAX₉₃YYCSRDPTEAPFDYWG QGX₁₁₂X₁₁₃VTVSS (SEQ ID NO: 54), where X₁₆ is G or R, X₁₉ is K or R, X₂₃ is A or V, X₃₅ is S or T, X₃₇ is I or V, X₆₁ is A, P or V, X₇₄ is D or N, X₇₅ is A or S, X₇₇ is N or S, X₇₈ is S or T, X₈₈ is A or S, X₉₃ is T or V, X₁₁₂ is T or V, and X₁₁₃ is L or M. For example, it may be divided according to Chothia rules.

In some cases, compared with an amino acid sequence as set forth in SEQ ID NO: 20, the VH may comprise at least amino acid substitutions at positions selected from the group consisting of amino acid substitutions at X₁₆, X₁₉, X₂₃, X₃₅, X₃₇, X₆₁, X₇₄, X₇₅, X₇₇, X₇₈, X₈₈, X₉₃, X₁₁₂ and/or X₁₁₃.

For example, the VH may comprise an amino acid sequence as set forth in any one of SEQ ID NO: 20 to 25.

In the present application, the isolated antigen-binding protein may comprise CDR3 frome a light chain variable region VL, and the VL may comprise an amino acid sequence as set forth in SEQ ID NO: 59.

In the present application, the isolated antigen-binding protein may comprise LCDR3, and the LCDR3 may comprise CDR3 of VL with an amino acid sequence as set forth in any one of SEQ ID NO: 36 to 39. For example, the isolated antigen-binding protein may comprise LCDR3, and the LCDR3 may comprise an amino acid sequence as set forth in SEQ ID NO: 6.

In the present application, the isolated antigen-binding protein may comprise CDR2 frome a light chain variable region VL, and the VL may comprise an amino acid sequence as set forth in SEQ ID NO: 59.

In the present application, the isolated antigen-binding protein may comprise LCDR2, and the LCDR2 may comprise CDR2 of VL with an amino acid sequence as set forth in any one of SEQ ID NO: 36 to 39. For example, the isolated antigen-binding protein may comprise LCDR2, and the LCDR2 may comprise an amino acid sequence as set forth in SEQ ID NO: 5.

In the present application, the isolated antigen-binding protein may comprise CDR1 from a light chain variable region VL, and the VL may comprise an amino acid sequence as set forth in SEQ ID NO: 59.

In the present application, the isolated antigen-binding protein may comprise LCDR1, and the LCDR1 may comprise CDR1 of VL with an amino acid sequence as set forth in any one of SEQ ID NO: 36 to 39. For example, the isolated antigen-binding protein may comprise LCDR1, and the LCDR1 may comprise an amino acid sequence as set forth in SEQ ID NO: 4.

In the present application, the isolated antigen-binding proteins may comprise LCDR1, LCDR2 and LCDR3, the LCDR1 may comprise CDR1 of VL with an amino acid sequence as set forth in SEQ ID NO: 59, the LCDR2 may comprise CDR2 of VL with an amino acid sequence as set forth in SEQ ID NO: 59, and the LCDR3 may comprise CDR3 of VL with an amino acid sequence as set forth in SEQ ID NO: 59.

In the present application, the isolated antigen-binding proteins may comprise LCDR1, LCDR2 and LCDR3, the LCDR1 may comprise CDR1 of VL with an amino acid sequence as set forth in any one of SEQ ID NO: 36 to 39, the LCDR2 may comprise CDR2 of VL with an amino acid sequence as set forth in any one of SEQ ID NO: 36 to 39, and the LCDR3 may comprise CDR3 of VL with an amino acid sequence as set forth in any one of SEQ ID NO: 36 to 39.

For example, the isolated antigen-binding protein may comprise CDR1 of VL with an amino acid sequence as set forth in SEQ ID NO: 36, may comprise CDR2 of VL with an amino acid sequence as set forth in SEQ ID NO: 36, and may comprise CDR3 of VL with an amino acid sequence as set forth in SEQ ID NO: 36.

For example, the isolated antigen-binding protein may comprise CDR1 of VL with an amino acid sequence as set forth in SEQ ID NO: 37, may comprise CDR2 of VL with an amino acid sequence as set forth in SEQ ID NO: 37, and may comprise CDR3 of VL with an amino acid sequence as set forth in SEQ ID NO: 37.

For example, the isolated antigen-binding protein may comprise CDR1 of VL with an amino acid sequence as set forth in SEQ ID NO: 38, may comprise CDR2 of VL with an amino acid sequence as set forth in SEQ ID NO: 38, and may comprise CDR3 of VL with an amino acid sequence as set forth in SEQ ID NO: 38.

For example, the isolated antigen-binding protein may comprise CDR1 of VL with an amino acid sequence as set forth in SEQ ID NO: 39, may comprise CDR2 of VL with an amino acid sequence as set forth in SEQ ID NO: 39, and may comprise CDR3 of VL with an amino acid sequence as set forth in SEQ ID NO: 38.

For example, the isolated antigen-binding proteins described in the present application may comprise LCDR1, LCDR2 and LCDR3, the LCDR1 may comprise an amino acid sequence as set forth in SEQ ID NO: 4, the LCDR2 may comprise an amino acid sequence as set forth in SEQ ID NO: 5, and the LCDR3 may comprise an amino acid sequence as set forth in SEQ ID NO: 6.

In the present application, the isolated antigen-binding protein may comprise L-FR1, and the L-FR1 may comprise an amino acid sequence as set forth in SEQ ID NO: 55: DIQMTQSPX₉SLSASX₁₅GDX₁₈VTITC (SEQ ID NO: 55), where X₉ is P or S, X₁₅ is L or V, and X₁₈ is Q or R. For example, it may be divided according to Chothia rules.

In some cases, compared with an amino acid sequence as set forth in SEQ ID NO: 26, the L-FR1 may comprise at least amino acid substitutions at positions selected from the group consisting of amino acid substitutions at X₉, X₁₅ and/or X₁₈.

For example, the L-FR1 may comprise an amino acid sequence as set forth in SEQ ID NO: 26 or 27.

In the present application, the isolated antigen-binding protein may comprise L-FR2, and the L-FR2 may comprise an amino acid sequence as set forth in SEQ ID NO: 56: WYQQKPGKAPX₁₁QLIR (SEQ ID NO: 56), where X₁₁ is K or R. For example, it may be divided according to Chothia rules.

In some cases, compared with an amino acid sequence as set forth in SEQ ID NO: 28, the H-FR2 may comprise at least amino acid substitutions at positions selected from the group consisting of amino acid substitutions at X₁₁.

For example, the L-FR2 may comprise an amino acid sequence as set forth in SEQ ID NO: 28 or 29.

In the present application, the isolated antigen-binding protein may comprise L-FR3, and the L-FR3 may comprise an amino acid sequence as set forth in SEQ ID NO: 57: GX₂PSRFSGSGSGKDX₁₅X₁₆FX₁₈ISX₂₁X₂₂X₂₃X₂₄EDIAX₂₉YYC (SEQ ID NO: 57), where X₂ is T or V, X₁₅ is F or Y, X₁₆ is SorT, X₁₈ is S or T, X₂₁ is N or S, X₂₂ is L or V, X₂₃ is E or Q, X₂₄ is P or S, and X₂₉ is S or T. For example, it may be divided according to Chothia rules.

In some cases, compared with an amino acid sequence as set forth in SEQ ID NO: 30, the L-FR3 may comprise at least amino acid substitutions at positions selected from the group consisting of amino acid substitutions at X₂, X₁₅, X₁₆, X₁₈, X₂₁, X₂₂, X₂₃, X₂₄ and/or X₂₉.

For example, the L-FR3 may comprise an amino acid sequence as set forth in any one of SEQ ID NO: 30 to 33.

In the present application, the isolated antigen-binding protein may comprise L-FR4, and the L-FR4 may comprise an amino acid sequence as set forth in SEQ ID NO: 58: FGX₃GTKLEX₉K (SEQ ID NO: 58), where X₃ is A or Q and X₉ is I or L. For example, it may be divided according to Chothia rules.

In some cases, compared with an amino acid sequence as set forth in SEQ ID NO: 34, the L-FR4 may comprise at least amino acid substitutions at positions selected from the group consisting of amino acid substitutions at X₃ and/or X₉.

For example, the L-FR4 may comprise an amino acid sequence as set forth in SEQ ID NO: 34 or 35.

In the present application, the isolated antigen-binding protein may comprise a light chain variable region VL, and the VL may comprise an amino acid sequence as set forth in SEQ ID NO: 59:
DIQMTQSPX₉SLSASX₁₅GDX₁₈VTITCQASQNINNYIAWYQQKPGKAPX₄₅QLIRYTSTLVSGX_{5 8}PSRFSGSGSGKDX₇₁X₇₂FX₇₄ISX₇₇X₇₈X₇₉X₈₀EDIAX₈₅YYCLQYDNVPNTFGX₁₀₀GTKLEX₁₀₆K (SEQ ID NO: 59), where X₉ is P or S, X₁₅ is L or V, X₁₈ is Q or R, X₄₅ is K or R, X₅₈ is T or V, X₇₁ is F or Y, X₇₂ is S or T, X₇₄ is T or S, X₇₇ is N or S, X₇₈ is L or V, X₇₉ is E or Q, X₈₀ is P or S, X₈₅ is S or T, X₁₀₀ is A or Q, and X₁₀₆ is L or I. For example, it may be divided according to Chothia rules.

In some cases, compared with an amino acid sequence as set forth in SEQ ID NO: 36, and the VL may comprise at least amino acid substitutions at positions selected from the group consisting of X₉, X₁₅, X₁₈, X₄₅, X₅₈, X₇₁, X₇₂, X₇₄, X₇₇, X₇₈, X₇₉, X₈₀, X₈₅, X₁₀₀ and/or X₁₀₆.

For example, the VL may comprise an amino acid sequence as set forth in any one of SEQ ID NO: 36 to 39.

In the present application, the isolated antigen-binding protein may comprise HCDR1, HCDR2, HCDR3, LCDR1, LCDR2 and LCDR3, and the HCDR1 may comprise an amino acid sequence as set forth in SEQ ID NO: 1, and the HCDR2 may comprise an amino acid sequence as set forth in SEQ ID NO: 2, the HCDR3 may comprise an amino acid sequence as set forth in SEQ ID NO: 3, the LCDR1 may comprise an amino acid sequence as set forth in SEQ ID NO: 4, the LCDR2 may comprise an amino acid sequence as set forth in SEQ ID NO: 5, and the LCDR3 may comprise an amino acid sequence as set forth in SEQ ID NO: 6.

In the present application, the isolated antigen-binding protein may comprise VH and VL, the VH may comprise an amino acid sequence as set forth in SEQ ID NO: 54, and the VL may comprise an amino acid sequence as set forth in SEQ ID NO: 59.

In the present application, the isolated antigen-binding protein may comprise VH and VL, the VH may comprise an amino acid sequence as set forth in any one of SEQ ID NO: 20 to 25, and the VL may comprise an amino acid sequence as set forth in any one of SEQ ID NO: 36 to 39.

For example, the isolated antigen-binding protein may comprise VH and VL, the VH may comprise an amino acid sequence as set forth in SEQ ID NO: 20, and the VL may comprise an amino acid sequence as set forth in SEQ ID NO: 36.

For example, the isolated antigen-binding protein may comprise VH and VL, the VH may comprise an amino acid sequence as set forth in SEQ ID NO: 21, and the VL may comprise an amino acid sequence as set forth in SEQ ID NO: 37.

For example, the isolated antigen-binding protein may comprise VH and VL, the VH may comprise an amino acid sequence as set forth in SEQ ID NO: 22, and the VL may comprise an amino acid sequence as set forth in SEQ ID NO: 38.

For example, the isolated antigen-binding protein may comprise VH and VL, the VH may comprise an amino acid sequence as set forth in SEQ ID NO: 23, and the VL may comprise an amino acid sequence as set forth in SEQ ID NO: 37.

For example, the isolated antigen-binding protein may comprise VH and VL, the VH may comprise an amino acid sequence as set forth in SEQ ID NO: 24, and the VL may comprise an amino acid sequence as set forth in SEQ ID NO: 37.

For example, the isolated antigen-binding protein may comprise VH and VL, the VH may comprise an amino acid sequence as set forth in SEQ ID NO: 25, and the VL may comprise an amino acid sequence as set forth in SEQ ID NO: 39.

In the present application, the isolated antigen-binding protein may comprise a heavy chain constant region, which may be derived from IgG. For example, the heavy chain constant region may be derived from human IgG. the heavy chain constant region may be derived from IgG1, IgG2, IgG3 and IgG4. For example, the heavy chain constant region may be derived from human IgG1. Compared with natural human IgG1, the heavy chain constant region may be amino acid mutated. For example, the heavy chain constant region may be a sequence obtained by performing N297A amino acid point mutation on IgG1. For example, the heavy chain constant region may comprise an amino acid sequence as set forth in SEQ ID NO: 41.

In the present application, the isolated antigen-binding protein may comprise a heavy chain, and the heavy chain may comprise an amino acid sequence as set forth in any one of SEQ ID NO: 42 to 46.

In the present application, the isolated antigen-binding protein may comprise a light chain constant region, and the light chain constant region may be derived from a light chain λ and a light chain κ. For example, the light chain constant region may comprise an amino acid sequence as set forth in SEQ ID NO: 40.

In the present application, the isolated antigen-binding protein may comprise a light chain, and the light chain may comprise an amino acid sequence as set forth in any one of SEQ ID NO: 47 to 49.

For example, the isolated antigen-binding protein may comprise a heavy chain and a light chain, the heavy chain may comprise an amino acid sequence as set forth in SEQ ID NO: 42, and the light chain may comprise an amino acid sequence as set forth in SEQ ID NO: 47.

For example, the isolated antigen-binding protein may comprise a heavy chain and a light chain, the heavy chain may comprise an amino acid sequence as set forth in SEQ ID NO: 43, and the light chain may comprise an amino acid sequence as set forth in SEQ ID NO: 48.

For example, the isolated antigen-binding protein may comprise a heavy chain and a light chain, the heavy chain may comprise an amino acid sequence as set forth in SEQ ID NO: 44, and the light chain may comprise an amino acid sequence as set forth in SEQ ID NO: 47.

For example, the isolated antigen-binding protein may comprise a heavy chain and a light chain, the heavy chain may comprise an amino acid sequence as set forth in SEQ ID NO: 45, and the light chain may comprise an amino acid sequence as set forth in SEQ ID NO: 47.

For example, the isolated antigen-binding protein may comprise a heavy chain and a light chain, the heavy chain may comprise an amino acid sequence as set forth in SEQ ID NO: 46, and the light chain may comprise an amino acid sequence as set forth in SEQ ID NO: 49.

In the present application, a part of the amino acid sequence of each heavy chain or light chain of the antigen-binding protein is homologous to the corresponding amino acid sequence in an antibody from a specific species, or belongs to a specific category. For example, the variable region and constant portion of light chain and heavy chain are both from the variable region and constant region of antibody of one animal species (such as human). In the present application, the homologue may be a protein and/or a polypeptide having at least about 85% (for example, at least about 85%, about 90%, about 91%, about 92%, about 93%, about 94%, about 95%, about 96%, about 97%, about 98%, about 99% or more) identity with the amino acid sequence of the protein and/or the polypeptide (for example, an antibody or fragment thereof that specifically binds to PD-L1 protein).

In the present application, the homology generally refers to similarity, likeness or association between two or more sequences. The alignment for determining the percentage of sequence homology can be realized in various ways known in the art, for example, using publicly available computer softwares such as BLAST, BLAST-2, ALIGN or Megalign (DNASTAR) softwares. Those skilled in the art may determine appropriate parameters for alignment sequences, including any algorithm needed to achieve maximum alignment within the full-length sequence being compared or within the target sequence area. The homology may also be determined by the following methods: FASTA and BLAST. For the description of FASTA algorithm, please refer to W. R. PearsonandD. J. Lipman, "Improved Tools for Biological Sequence Comparison", Proc. Natl. Acad. Sci., 85:2444-2448, 1988; and D. J. Lipman and W. R. Pearson, " Rapid and sensitive protein similarity searches", Science, 227:1435-1441, 1989. Descriptions of BLAST algorithm may be found in S. Altschul, W. Gish, W. Miller, E. W. Myers and D. Lipman, "Basic Local Alignment Search Tool", Journal of Molecular Biology, 215:403-410, 1990.

### Test Method

The physical/chemical properties and/or biological activities of the PD-1 antigen-binding protein described in the present application may be identified, screened or characterized by various assays known in the art.

In one aspect, for example, the antigen binding activity of the antigen-binding protein of the present application may be tested by known methods such as enzyme-linked immunosorbent assay (ELISA), western blot (e.g., protein blot), flow cytometry (e.g., FACS), immunohistochemistry, immunofluorescence or the like.

### Nucleic acid, vector, host cell and preparation method

In another aspect, the present application also provides one or more isolated nucleic acid molecules. The one or more nucleic acid molecules may encode the antigen-binding protein described herein. For example, each nucleic acid molecule in the one or more nucleic acid molecules may encode the complete antigen-binding protein, or may encode a portion thereof (for example, one or more of HCDR1-3, LCDR1-3, VL, VH, a light chain or a heavy chain).

The nucleic acid molecule described in the present application may be isolated. For example, it may be produced or synthesized by the following methods: (i) amplification *in vitro,* for example, polymerase chain reaction (PCR) amplification, (ii) cloning and recombination, (iii) purification, for example, fractionation by enzyme digestion and gel electrophoresis, or (iv) synthesis, for example, chemical synthesis. In some embodiments, the isolated nucleic acid is a nucleic acid molecule prepared by recombinant DNA technology.

In the present application, the nucleic acid encoding the antibody and antigen-binding fragment thereof may be prepared by various methods known in the art, including but not limited to restriction fragment manipulation or overlapping extension PCR using synthetic oligonucleotides.

In another aspect, the present application provides one or more vectors comprising one or more nucleic acid molecules described herein. Each vector may comprise one or more of the nucleic acid molecules. In addition, the vector may also comprise other genes, such as marker genes that allow selection of the vector in appropriate host cells and under appropriate conditions. In addition, the vector may also comprise an expression control element that allows the coding region to be correctly expressed in an appropriate host. For example, the vector is an expression vector.

In another aspect, the present application provides a host cell, and the host cell may comprise one or more nucleic acid molecules and/or one or more vectors described herein. In some embodiments, each or every host cell may comprise one or more nucleic acid molecules or vectors described herein. In some embodiments, each or every host cell may comprise a plurality (e.g., two or more) or a variety (e.g., two or more kinds) of nucleic acid molecules or vectors described herein.

In another aspect, the present application provides a method for preparing the antibody or antigen-binding fragment thereof. The method may comprise culturing the host cell described in the present application under conditions such that the antibody or antigen-binding fragment thereof is expressed. For example, with suitable culture medium, suitable temperature and culture time, etc., these methods are known to those skilled in the art.

### Pharmaceutical composition. Method and Use

In another aspect, the present application provides a pharmaceutical composition. The pharmaceutical composition may comprise the antigen-binding protein, the polypeptide, the nucleic acid molecule, the vector, the host cell described herein, and optionally a pharmaceutically acceptable carrier. The pharmaceutically acceptable adjuvant is not toxic to the recipient at the dosage and concentration adopted, and the pharmaceutical composition in the present application may also comprise more than one active compounds, which are those active compounds with complementary activities and usually do not adversely affect each other. The form and effective amount of such pharmaceuticals depend on, for example, the amount and form of antagonists present in the preparation, and the clinical parameters of the subject.

The pharmaceutical composition may be used for inhibiting tumor growth. For example, the pharmaceutical composition of the present application may inhibit or delay the development or progression of a disease, reduce the tumor size (even basically eliminate the tumor), and/or alleviate and/or stabilize the disease conditions.

The pharmaceutical composition of the present application may comprise a prophylactically and/or therapeutically effective amount of the antibody, antigen-binding fragment thereof. The prophylactically and/or therapeutically effective amount is the dosage that is required to prevent and/or treat (at least partially treat) a disease or disorder and/or any complications thereof in a subject having or at risk of developing the disease or disorder.

In another aspect, the present application provides a use of the antigen-binding protein and/or the fusion protein in the manufacture of a medicament. The medicament is for use in treating cancer, inhibiting tumor growth and/or inhibiting tumor cell proliferation. In some embodiments, the tumor or cancer is a tumor or cancer with abnormal expression of PD-L1. In the present application, the tumor may include a tumor with a high expression of PD-1 or PD-L1. In the present application, the tumor may include a tumor with a high expression of PD-L1. In the present application, the tumor may include a solid tumor. In the present application, the tumor may include breast cancer, lung cancer, gastric cancer and urothelial carcinoma.

In another aspect, the present application provides a method for inhibiting the binding of PD-L1 to PD-1, which comprises administering the antigen-binding protein and/or the polypeptide described herein. For example, the method may be *ex vivo* or *in vitro.* For example, the method may be a method for non-therapeutic purposes. In some cases, the method may comprise contacting a biological sample with the antigen-binding protein and/or PD-1 described herein under conditions that allow the antigen-binding protein and/or PD-1 to bind to PD-L1, detecting whether a complex is formed between the antigen-binding protein and PD-L1, and detecting whether a complex is formed between PD-1 and PD-L1.

In another aspect, the present application provides a method for stimulating the secretion of cytokines in immune cells, which comprises administering the isolated antigen-binding protein and/or the polypeptide. The cytokine may be IL-2. The immune cells may be lymphocytes. For example, T lymphocytes. For example, the method may be *ex vivo* or *in vitro.* For example, the method may be a method for non-therapeutic purpose.

In another aspect, the present application provides a method for detecting the presence and/or content of PD-L1 protein, which comprises administering the isolated antigen-binding protein and/or the polypeptide. For example, the method can be *ex vivo* or *in vitro.* For example, the method may be a method for non-therapeutic purposes.

The present application also provides the use of antigen-binding protein in a method for diagnosing a subject suffering from a tumor or cancer, the method comprises: determining the presence or expression level of PD-L1 in a sample obtained from the subject by contacting the sample with the antigen-binding protein of the present application and detecting the presence of the bound antibody.

In another aspect, the present application provides a chimeric antigen receptor (CAR), which may comprise the nucleic acid molecule or antigen-binding protein described in the present application.

In another aspect, the present application provides an antibody drug conjugate, which may include the antigen-binding fragment.

In another aspect, the present application provides a kit, which may comprise the antigen-binding protein, the chimeric antigen receptor, the genetically modified cell, the antibody drug conjugate described in the present application, and/or the pharmaceutical composition described in the present application. It may comprise the antigen-binding protein, the chimeric antigen receptor, the genetically modified cell described in the present application, and/or the antibody drug conjugate described in the present application in a single common container, and may also be optionally in combination with one or more therapeutic agents, and optionally formulated together in a pharmaceutical compositions

In another aspect, the present application provides a drug delivery device, which may be used for administering the antigen-binding protein or a pharmaceutical composition thereof.

Without being bound by any theory, the following examples are only for explaining various embodiments of the invention, and they are not used to limit the scope of the invention.

### Example

### Example 1. Preparation of Murine Anti-Human PD-L1 Monoclonal Antibody

Emulsification was carried out with Human PD-L1B7-H1 Protein, Fc Tag (Acro Biosystem PD1-H5258) and PADRE, and then 6 to 8-week-old female SD rats were injected intraperitoneally with 50 µg Human PD-L1B7-H1 Protein, Fc Tag/rat, 25 µg PADRE/rat; after that, booster immunization was carried out at intervals of two to three weeks. The rats were immunized three times in total, and two weeks after the last immunization, the tail blood of rats was taken to determine the titer of anti-human PDL1 antibody in serum. After 50 µg protein without Freund's adjuvant was injected intraperitoneally into the rats for ictus immunisatorius, the spleen cells of the rats were taken and fused with myeloma (Sp2/0) cells on the third day.

Clones that specifically bind to HEK293-PD-L1 cells and can simultaneously block the binding of receptor PD-1 to HEK293-hPD-L1 cells were screened from the fusion plate by flow cytometer and subcloning was carried out by limited dilution method, and finally a monoclonal that can secrete specific antibodies was screened. Monoclonal antibodies were obtained by small-scale production and purification in serum-free medium for subsequent identification, including the detection of the binding ability of antibodies to HEK293-PD-L1, the function of blocking binding of PD-1 to HEK293-hPD-L1 cell, and the mixed lymphocyte experiment to finally obtain candidate anti-human PD-L1 monoclonal antibody 50G2-3. The sequences of the variable regions of murine hybridoma clone 50G2-3 obtained by monoclonal antibody sequencing is as follows:
> 50G2-3 light chain variable region sequence 
DIQMTQSPPSLSASLGDQVTITCQASQNINNYIAWYQQKPGKAPRQLIRYTSTLVSGTPS
RFSGSGSGKDYSFSISNVESEDIASYYCLQYDNVPNTFGAGTKLELK (SEQ ID NO: 36)
>50G2-3 heavy chain variable region sequence

EVQLVESGGGLVQPGRSLKLSCVASGFTFSNFWMTWIRQAPGKGLEWVASITHSGGIT
YYPDSVKGRFTISRDDAKSTLYLQMNSLRSEDTATYYCSRDPTEAPFDYWGQGVMVTVSS
(SEQ ID NO: 20)

**Table 1. Heavy Chain and Light Chain CDR Sequences of Murine Hybridoma Clone 50G2-3**

| Heavy Chain | | Light Chain | |
|---|---|---|---|
| HCDR1 | GFTFSNF (SEQ ID NO: 1) | LCDR1 | QASQNINNYIA (SEQ ID NO: 4) |
| HCDR2 | THSGGI (SEQ ID NO: 2) | LCDR2 | YTSTLVS (SEQ ID NO: 5) |
| HCDR3 | DPTEAPFDY (SEQ ID NO: 3) | LCDR3 | LQYDNVPNT (SEQ ID NO: 6) |

### Example 2. Humanization of Monoclonal Antibodies

The most homologous human Germline antibody (data source: IMGT) was selected as the humanized design framework (the framework of the light chain was based on IGKV1-33*01, IGKJ2*01, and the framework of the heavy chain was based on IGHV3-7*01, IGHJ4*01), and the variable regions of the light and heavy chains of the antibody were numbered by Chothia [Chothia & Lesk, 1987], and CDR regions of the antibody were defined as: CDRL1(L24-L34), CDRL2(L50-L56), CDRL3(L89-L97), CDRH1(H26-H32), CDRH2(H52-H56) and CDRH3(H95-H97), and humanized mutations of amino acids of light and heavy chain variable regions of antibodies were carried out according to sequence alignment and structural information of variable regions; expression vectors were designed, genes were synthesized, recombinant antibodies were expressed by mammalian cells and purified, the differences in activity and physical and chemical properties between humanized antibodies and chimeric antibodies were compared, and 1 to 2 rounds of humanization optimization were carried out.

Germline antibody sequence information:
➢ IGKV1-33*01 
DIQMTQSPSSLSASVGDRVTITCQASQDISNYLNWYQQKPGKAPKLLIYDASNLETGVP
SRFSGSGSGTDFTFTISSLQPEDIATYYCQQYDNLP (SEQ ID NO: 60)
➢ IGKJ2*01
   YTFGQGTKLEIK (SEQ ID NO: 61)
➢ IGHV3-7*01 
EVQLVESGGGLVQPGGSLRLSCAASGFTFSSYWMSWVRQAPGKGLEWVANIKQDGSE
KYYVDSVKGRFTISRDNAKNSLYLQMNSLRAEDTAVYYCAR (SEQ ID NO: 62)
➢ IGHJ4*01
   YFDYWGQGTLVTVSS (SEQ ID NO: 63)

The following optimized designs of light and heavy chains were humanized sequences transplanted based on the sequence of the above Germanline antibody as framework CDRs (1910G2HzL0 and 1910G2HzH0 are the light and heavy chains of the chimeric antibody, and 1910G2HzL2, 1910G2HzH2, 1910G2HzL3, 1910G2HzH3, 1910G2HzL4, 1910G2HzH4, 1910G2HzL7, 1910G2HzH7, 1910G2HzL9 and 1910G2HzH9 are the light and heavy chains of the humanized antibody).
➢ 1910G2HzL0 light chain variable region sequence 
DIQMTQSPPSLSASLGDQVTITCQASQNINNYIAWYQQKPGKAPRQLIRYTSTLVSGTPS
RFSGSGSGKDYSFSISNVESEDIASYYCLQYDNVPNTFGAGTKLELK (SEQ ID NO: 36)
➢ 1910G2HzL2 light chain variable region sequence 
DIQMTQSPSSLSASVGDRVTITCQASQNINNYIAWYQQKPGKAPKQLIRYTSTLVSGVP
SRFSGSGSGKDYTFTISSLQPEDIATYYCLQYDNVPNTFGQGTKLEIK (SEQ ID NO: 37)
➢ 1910G2HzL3 light chain variable region sequence 
DIQMTQSPSSLSASVGDRVTITCQASQNINNYIAWYQQKPGKAPKQLIRYTSTLVSGTPS
RFSGSGSGKDYTFTISNLQPEDIATYYCLQYDNVPNTFGQGTKLEIK (SEQ ID NO: 38)
➢ 1910G2HzL4 light chain variable region sequence
   Same as 1910G2HzL2 (SEQ ID NO: 37)
➢ 1910G2HzL7 light chain variable region sequence
   Same as 1910G2HzL2 (SEQ ID NO: 37)
➢ 1910G2HzL9 light chain variable region sequence 
DIQMTQSPSSLSASVGDRVTITCQASQNINNYIAWYQQKPGKAPKQLIRYTSTLVSGVP
SRFSGSGSGKDFTFTISSLQPEDIATYYCLQYDNVPNTFGQGTKLEIK (SEQ ID NO: 39)
➢ 1910G2HzH0 heavy chain variable region sequence 
EVQLVESGGGLVQPGRSLKLSCVASGFTFSNFWMTWIRQAPGKGLEWVASITHSGGIT
YYPDSVKGRFTISRDDAKSTLYLQMNSLRSEDTATYYCSRDPTEAPFDYWGQGVMVTVSS
(SEQ ID NO: 20)
➢ 1910G2HzH2 heavy chain variable region sequence 
EVQLVESGGGLVQPGGSLRLSCAASGFTFSNFWMTWVRQAPGKGLEWVASITHSGGIT
YYVDSVKGRFTISRDDAKSSLYLQMNSLRAEDTAVYYCSRDPTEAPFDYWGQGTLVTVSS
(SEQ ID NO: 21)
➢ 1910G2HzH3 heavy chain variable region sequence 
EVQLVESGGGLVQPGGSLRLSCAASGFTFSNFWMSWVRQAPGKGLEWVASITHSGGIT
YYVDSVKGRFTISRDNAKSSLYLQMNSLRAEDTAVYYCSRDPTEAPFDYWGQGVMVTVSS
(SEQ ID NO: 22)
➢ 1910G2HzH4 heavy chain variable region sequence 
EVQLVESGGGLVQPGGSLRLSCAASGFTFSNFWMTWVRQAPGKGLEWVASITHSGGIT
YYPDSVKGRFTISRDDSKNTLYLQMNSLRAEDTAVYYCSRDPTEAPFDYWGQGTLVTVSS
(SEQ ID NO: 23)
➢ 1910G2HzH7 heavy chain variable region sequence 
EVQLVESGGGLVQPGGSLRLSCAASGFTFSNFWMTWVRQAPGKGLEWVASITHSGGIT
YYADSVKGRFTISRDDSKSTLYLQMNSLRAEDTAVYYCSRDPTEAPFDYWGQGTLVTVSS
(SEQ ID NO: 24)
➢ 1910G2HzH9 heavy chain variable region sequence

EVQLVESGGGLVQPGGSLRLSCAASGFTFSNFWMSWVRQAPGKGLEWVASITHSGGIT
YYADSVKGRFTISRDDSKNTLYLQMNSLRAEDTAVYYCSRDPTEAPFDYWGQGTLVTVSS
(SEQ ID NO: 25)

The light chains and heavy chains of the antibodies described above are combined to obtain antigen-binding proteins 1910G2HzL2H2, 1910G2HzL3H3, 1910G2HzL4H4, 1910G2HzL7H7 and 1910G2HzL9H9.

### Example 3. Determination of Kinetic Parameters of the Antigen-binding protein of the Present Application

(1) The affinities of humanized antibodies 1910G2HzL2H2, 1910G2HzL3H3, 1910G2HzL4H4, 1910G2HzL7H7 and 1910G2HzL9H9 to human PD-L1 (Aero, Catalog Number: H5229) were determined by Octet RED96e (Fortebio), both antigens and antibodies were diluted by 1xPBST (1xPBS: Sangon, B548117-0500; 0.02% Tween 20: Sigma-Alorich, P1379), and the concentration of antigen for use was 100 nM, and the concentration of antibody for use was 50 nM.
(2) Detection of samples on machines (Octet Data Acquisition 11.1.0.11): First, the sample was added into a 96-well plate (Greiner bio-one, 655209) with a system of 200 µL/well. Then the software parameters were set, the plate temperature was set to 30°C, and the frequency of collecting standard dynamic signals was 5.0 HZ. Next, the AHC sensor (Fortébio, Catalog Number: 18-0015) was prewetted with 1xPBST for 10 minutes, and then the sample was detected on the instrument. Each cycle incluedes the following steps: 1) immersing in buffer for 60 seconds; 2) detecting whether the antigen nonspecifically binding to the sensor; 3) regeneration of 10 mM glycine solution with pH 1.7; 4) immersing in the buffer for 60 seconds; 5) immobilizing the antibody on the sensor for 20 seconds; 6) immersing the sensor in the buffer for 180 seconds; 7) the antigen binding to the antibody for 180 seconds; 8) dissociating the antigen from the antibody for 10 minutes; 9) regenerating the sensor.
(3) Data analysis

The association rate (Ka) and dissociation rate (Kd) of the antigen-antibody at a ratio of 1:1 were measured by Data Analysis 11.0 software of Fortebio, and based on these, the equilibrium dissociation constant (KD) of antibodies were calculated. The results are as shown in the Table below, indicating that the affinities of humanized antibodies 1910G2HzL2H2, 1910G2HzL3H3, 1910G2HzL4H4, 1910G2HzL7H7 and 1910G2HzL9H9 to human PD-L1 are comparable to Atezolimumab, and even with lower KD values. Theses indicated that the antigen-binding proteins of the present application had higher binding affinities.

**Table 2. Affinity of Humanized Antibodies to Human PD-L1**

| Humanized Antibody | KD (M) | kon (1/Ms) | kdis (1/s) |
|---|---|---|---|
| 1910G2HzL2H2 | 2.435E-10 | 1.03E+06 | 2.50E-04 |
| 1910G2HzL3H3 | 4.522E-10 | 1.06E+06 | 4.77E-04 |
| 1910G2HzL4H4 | 1.601E-10 | 1.03E+06 | 1.65E-04 |
| 1910G2HzL7H7 | 3.019E-10 | 9.58E+05 | 2.89E-04 |
| 1910G2HzL9H9 | 2.137E-10 | 9.80E+05 | 2.10E-04 |
| Atezolimumab | 4.837E-10 | 6.44E+05 | 3.11E-04 |

### Example 4. Binding Activity of the Antigen-binding protein of the present application to Antigen PD-L1 on the Cell Surface.

CHOK1-cynoPD-L1 or HEK293-hPD-L1 were collected with 5E6 cells in each 96-well plate, centrifuged at 300g for 5 minutes, resuspended with pre-cooled FACS buffer, evenly spread on a 3799 U-shaped plate by 100µL/well, and blocked at room temperature for 15 minutes. Humanized antibodies were taken, started at an initial concentration of 3.33 µg/mL, and then diluted three-fold to establish 12 gradients. After blocking and centrifugation at 2000rpm for 5 minutes, and the supernatant was removed. Diluted antibodies were added by 100 µL/well, and the mixture was mixed evenly, and incubated at 4°C for 1 hour. The mixture was centrifuged at 2000rpm for 5 minutes, the supernatant was removed, and the residue was washed with FACS Buffer for 1-2 times. A secondary antibody goat-anti-human488, 1:1000 was added by 100 µL/well, and incubated at 4°C for 1 hour, centrifuged at 2000 rpm for 5 minutes, then the supernatant was removed, and the residue was washed with FACS Buffer for 1-2 times, and then FACS buffer was added by 30 µl/well for resuspension of the cells and detection on the instrument.

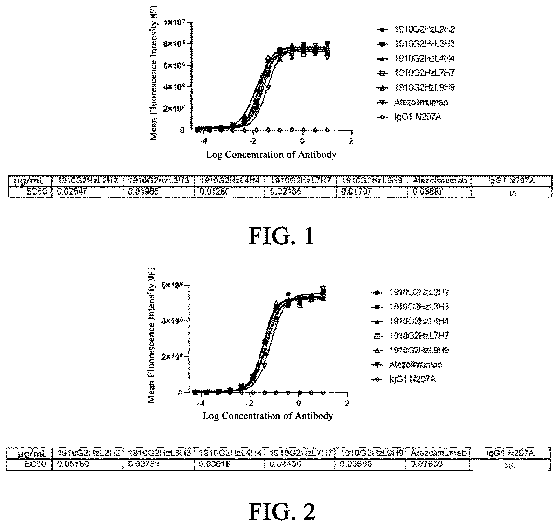

Fig. 1 showed that the antigen-binding protein described in the present application binds to the human PD-L1 protein on the HEK293 cell. Fig. 2 showed that the antigen-binding protein described in the present application binds to the monkey PD-L1 protein on the CHOK1 cell. Results were as shown in Fig. 1 and Fig. 2, and the binding activities of antigen-binding proteins 1910G2HzL2H2, 1910G2HzL3H3, 1910G2HzL4H4, 1910G2HzL7H7 and 1910G2HzL9H9 of the present application to antigen PD-L1 on the cell surface were comparable to or even higher than Atezolimumab. It was indicated that the binding activities of the antigen-binding proteins of the present application to PD-L1 were excellent.

### Example 5. The Antigen-binding protein of the Present Application Blocks the Binding Activity of Antigen PD-L1 on the Cell Surface to PD-1.

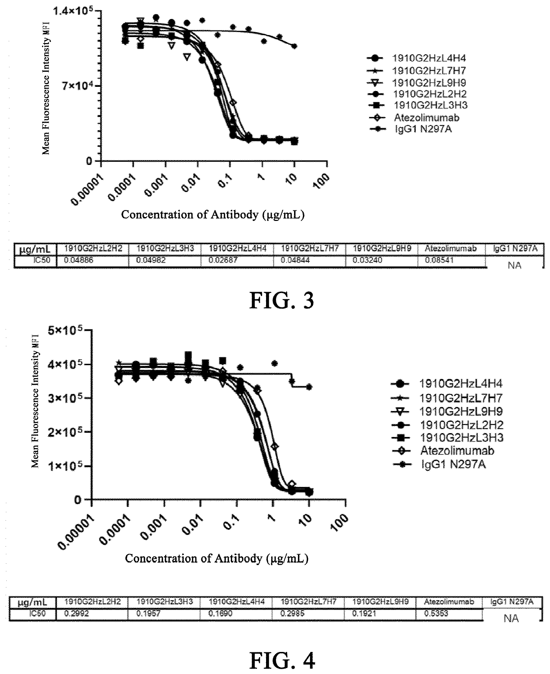

HEK293-hPD-L1 or CHOK1-cynoPD-L1 were collected by 5E6 cells in each 96-well plate, centrifuged at 300g for 5 minutes, resuspended with pre-cooled FACS buffer, evenly spread on a 3799 U-shaped plate by 100µL/well, and blocked at room temperature for 15 minutes. Humanized antibodies were taken and started at an initial concentration of 10 µg/mL, and then diluted three-fold to obtain 12 gradients. Biotin-PD-1-mFc of 0.224 mg/mL was prepared into 2 µg/mL. After blocking and centrifugation at 2000rpm for 5 minutes, and the supernatant was removed. Diluted antibodies were added by 50 µL/well, the mixtured was mixed evenly, and 2 µg/mL of Biotin-PD-1-mFc was added by 50µL/well, mixed evenly, and incubated at 4°C for 1 hour. After a centrifugation at 2000 rpm for 5 minutes, the supernatant was removed, and the residue was washed with FACS Buffer for 1-2 times. A secondary antibody SA488,1:1000 was added by 100 µL/well, and incubated at 4°C for 1 hour, centrifuged at 2000 rpm for 5 minutes, then the supernatant was removed. The residue was washed with FACS Buffer for 1-2 times, and then FACS buffer was added by 30 µl/well for resuspension of the cells and detection on the instrument. Fig. 3 showed that the antigen-binding protein described in the present application blocked the binding of PD-1 to the human PD-L1 protein on the HEK293 cell. Fig. 4 showed that the antigen-binding protein described in the present application blocked the binding of PD-1 to the monkey PD-L1 protein on the CHOK1 cell. Results were as shown in Fig. 3 and Fig. 4, and the activities of antigen binding proteins 1910G2HzL2H2, 1910G2HzL3H3, 1910G2HzL4H4, 1910G2HzL7H7 and 1910G2HzL9H9 of the present application to block the binding of antigen PD-L1 on the cell surface to PD-1 were comparable to Atezolimumab. It was indicated that the activities of the antigen-binding proteins of the present application to block the binding of PD-L1 to PD-1 were excellent.

### Example 6. Mixed Lymphocyte Reaction: Secretion of Cytokine IL-2

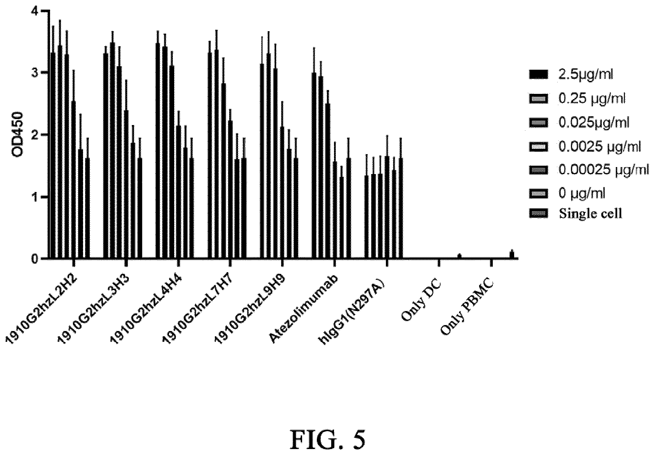

Human dendritic DC cells were resuscitated with cell culture medium (1640+ 2% FBS), the density of DC cells was adjusted to 1*10⁵ to 1*10⁷ cells /mL, then mitomycin C was added with a final concentration of 50 µg/ml, treating at 37 degrees in the dark for 30 minutes, then 10 mL culture medium was added for termination; the mixture was centrifuged at 400g for 10 minutes, and then washed with 10 mL culture medium. Gradient dilution of anti-PD1 antibody: the greatest final concentration of antibody was 2.5 µg/mL (the preparation concentration was 10 µg/mL), and the gradient dilution was ten-fold (5 concentration points + one 0 concentration), and then 50 µL of prepared anti-PD1 antibody was added into the corresponding cell culture plate (Corning, Catalog Number: 3599). DC cells treated with human peripheral blood lymphocytes PBMC and mitomycin C were collected, the density of DC cells was adjusted to 2*10⁵ cells/mL, and then the cells were added into the culture plate by 50 µL/well, i.e., the number of DC cells per well was 1*10⁴ cells/well; the density of PBMC cells was adjusted to 2*10⁶ cells /mL, and then the cells were added into the culture plate by 100 µL/well, i.e., the number of PBMC cells per well was 2*10⁵ cells/well. The cell culture plate was placed in a cell incubator with 5% carbon dioxide at 37°C for 5 days. After 5 days, the supernatant was collected following a centrifugation with 300g for 5 minutes, and the content of IL-2 was detected with Human IL-2 ELISAkit (BD, Catalog Number: 550611). The detection method was strictly performed in accordance with the kit instructions, and the data was processed with GraphPad Prism software. The results were as shown in Fig. 5, indicating all of the antigen-binding proteins 1910G2HzL2H2, 1910G2HzL3H3, 1910G2HzL4H4, 1910G2HzL7H7 and 1910G2HzL9H9 of the present application can stimulate the lymphocytes to secrete cytokines, with better effects than Atezolimumab. It was indicated that the antigen-binding proteins of the present application had high ability to stimulate immune cells to secrete cytokines.

The foregoing detailed description is provided by way of explanation and example, and is not intended to limit the scope of the appended claims. Many variations of the embodiments enumerated in the present invention are obvious to those skilled in the art, and they are within the scope of the appended claims and their equivalents.

## Claims

1. An isolated antigen-binding protein having one or more of the following properties:
(1) capable of binding to PD-L1 protein derived from a primate with a *K_{D}* value of 1×10⁹M or less;
(2) capable of blocking the binding of PD-L1 protein to PD-1 protein; and
(3) capable of stimulating the secretion of cytokines in immune cells.

2. The isolated antigen-binding protein according to claim 1, wherein the primate includes a human and/or a monkey.

3. The isolated antigen-binding protein according to claim 1 or 2, including an antibody or an antigen-binding fragment thereof.

4. The isolated antigen-binding protein according to claim 3, wherein the antigen-binding fragment includes a Fab, a Fab', a F(ab)₂, a Fv fragment, a F(ab')₂, a scFv, a di-scFv, a VHH and/or a dAb.

5. The isolated antigen-binding protein according to any one of claims 3 to 4, wherein the antibody is selected from the group consisting of a monoclonal antibody, a chimeric antibody, a humanized antibody and a fully human antibody.

6. The isolated antigen-binding protein according to any one of claims 1 to 5, wherein the isolated antigen-binding protein competes with a reference antibody for binding to PD-L1 protein, wherein the reference antibody comprises a heavy chain variable region VH and a light chain variable region VL, the VH of the reference antibody comprises HCDR1, HCDR2 and HCDR3, the VL of the reference antibody comprises LCDR1, LCDR2 and LCDR3, and the HCDR1 of the reference antibody comprises an amino acid sequence as set forth in SEQ ID NO: 1, the HCDR2 of the reference antibody comprises an amino acid sequence as set forth in SEQ ID NO: 2, the HCDR3 of the reference antibody comprises an amino acid sequence as set forth in SEQ ID NO: 3, the LCDR1 of the reference antibody comprises an amino acid sequence as set forth in SEQ ID NO: 4, the LCDR2 of the reference antibody comprises an amino acid sequence as set forth in SEQ ID NO: 5, and the LCDR3 of the reference antibody comprises an amino acid sequence as set forth in SEQ ID NO: 6.

7. The isolated antigen-binding protein according to any one of claims 1 to 6, wherein the isolated antigen-binding protein comprises HCDR3, and the HCDR3 comprises an amino acid sequence as set forth in SEQ ID NO: 3.

8. The isolated antigen-binding protein according to any one of claims 1 to 7, wherein the isolated antigen-binding protein comprises HCDR2, and the HCDR2 comprises an amino acid sequence as set forth in SEQ ID NO: 2.

9. The isolated antigen-binding protein according to any one of claims 1 to 8, wherein the isolated antigen-binding protein comprises HCDR1, and the HCDR1 comprises an amino acid sequence as set forth in SEQ ID NO: 1.

10. The isolated antigen-binding protein according to any one of claims 1 to 9, comprising HCDR1, HCDR2 and HCDR3, wherein the HCDR1 comprises an amino acid sequence as set forth in SEQ ID NO: 1, the HCDR2 comprises an amino acid sequence as set forth in SEQ ID NO: 2, and the HCDR3 comprises an amino acid sequence as set forth in SEQ ID NO: 3.

11. The isolated antigen-binding protein according to any one of claims 1 to 9, comprising a heavy chain variable region VH, wherein the VH comprises a framework region H-FR1, and the C-terminus of the H-FR1 is directly or indirectly linked to the N-terminus of the HCDR1, and the H-FR1 comprises an amino acid sequence as set forth in SEQ ID NO: 50.

12. The isolated antigen-binding protein according to claim 11, wherein the H-FR1 comprises an amino acid sequence as set forth in any one of SEQ ID NO: 7 or SEQ ID NO: 8.

13. The isolated antigen-binding protein according to any one of claims 11 to 12, wherein the VH comprises a framework region H-FR2, the H-FR2 is located between the HCDR1 and the HCDR2, and the H-FR2 comprises an amino acid sequence as set forth in SEQ ID NO: 51.

14. The isolated antigen-binding protein according to claim 13, wherein the H-FR2 comprises an amino acid sequence as set forth in any one of SEQ ID NO: 9 to 11.

15. The isolated antigen-binding protein according to any one of claims 11 to 14, wherein the VH comprises a framework region H-FR3, the H-FR3 is located between the HCDR2 and the HCDR3, and the H-FR3 comprises an amino acid sequence as set forth in SEQ ID NO: 52.

16. The isolated antigen-binding protein according to claim 15, wherein the H-FR3 comprises an amino acid sequence as set forth in any one of SEQ ID NO: 12 to 17.

17. The isolated antigen-binding protein according to any one of claims 11 to 16, wherein the VH comprises a framework region H-FR4, the N-terminus of the H-FR4 is linked to the C-terminus of the HCDR3, and the H-FR4 comprises an amino acid sequence as set forth in SEQ ID NO: 53.

18. The isolated antigen-binding protein according to claim 17, wherein the H-FR4 comprises an amino acid sequence as set forth in SEQ ID NO: 18 or 19.

19. The isolated antigen-binding protein according to any one of claims 1 to 18, comprising H-FR1, H-FR2, H-FR3 and H-FR4, wherein the H-FR1 comprises an amino acid sequence as set forth in SEQ ID NO: 7 or 8, the H-FR2 comprises an amino acid sequence as set forth in any one of SEQ ID NO: 9 to 11, the H-FR3 comprises an amino acid sequence as set forth in any one of SEQ ID NO: 12 to 17 and the H-FR4 comprises an amino acid sequence as set forth in SEQ ID NO: 18 or 19.

20. The isolated antigen-binding protein according to any one of claims 1 to 19, comprising H-FR1, H-FR2, H-FR3 and H-FR4, and the H-FR1, H-FR2, H-FR3 and H-FR4 are selected from any one of groups of amino acid sequences as follows:
(1) H-FR1: SEQ ID NO: 7, H-FR2: SEQ ID NO: 9, H-FR3: SEQ ID NO: 12 and H-FR4: SEQ ID NO: 18;
(2) H-FR1: SEQ ID NO: 8, H-FR2: SEQ ID NO: 10, H-FR3: SEQ ID NO: 13 and H-FR4: SEQ ID NO: 19;
(3) H-FR1: SEQ ID NO: 8, H-FR2: SEQ ID NO: 11, H-FR3: SEQ ID NO: 14 and H-FR4: SEQ ID NO: 18;
(4) H-FR1: SEQ ID NO: 8, H-FR2: SEQ ID NO: 10, H-FR3: SEQ ID NO: 15 and H-FR4: SEQ ID NO: 19;
(5) H-FR1: SEQ ID NO: 8, H-FR2: SEQ ID NO: 10, H-FR3: SEQ ID NO: 16 and H-FR4: SEQ ID NO: 19; and
(6) H-FR1: SEQ ID NO: 8, H-FR2: SEQ ID NO: 11, H-FR3: SEQ ID NO: 17 and H-FR4: SEQ ID NO: 19.

21. The isolated antigen-binding protein according to any one of claims 1 to 20, wherein the isolated antigen-binding protein comprises a heavy chain variable region VH, and the VH comprises an amino acid sequence as set forth in SEQ ID NO: 54.

22. The isolated antigen-binding protein according to claim 21, wherein the VH comprises an amino acid sequence as set forth in any one of SEQ ID NO: 20 to 25.

23. The isolated antigen-binding protein according to any one of claims 1 to 22, wherein the isolated antigen-binding protein comprises a heavy chain constant region, and the heavy chain constant region is derived from a human IgG constant region.

24. The isolated antigen-binding protein according to claim 23, wherein the heavy chain constant region is derived from a human IgG1 constant region, and the human IgG1 constant region comprises an amino acid sequence as set forth in SEQ ID NO: 41.

25. The isolated antigen-binding protein according to any one of claims 1 to 24, wherein the isolated antigen-binding protein comprises an antibody heavy chain HC, and the HC comprises an amino acid sequence as set forth in any one of SEQ ID NO: 42 to 46.

26. The isolated antigen-binding protein according to any one of claims 1 to 25, wherein the isolated antigen-binding protein comprises LCDR3, and the LCDR3 comprises an amino acid sequence as set forth in SEQ ID NO: 6.

27. The isolated antigen-binding protein according to any one of claims 1 to 26, wherein the isolated antigen-binding protein comprises LCDR2, and the LCDR2 comprises an amino acid sequence as set forth in SEQ ID NO: 5.

28. The isolated antigen-binding protein according to any one of claims 1 to 27, wherein the isolated antigen-binding protein comprises LCDR1, and the LCDR1 comprises an amino acid sequence as set forth in SEQ ID NO: 4.

29. The isolated antigen-binding protein according to any one of claims 1 to 28, comprising LCDR1, LCDR2 and LCDR3, wherein the LCDR1 comprises an amino acid sequence as set forth in SEQ ID NO: 4, the LCDR2 comprises an amino acid sequence as set forth in SEQ ID NO: 5, and the LCDR3 comprises an amino acid sequence as set forth in SEQ ID NO: 6.

30. The isolated antigen-binding protein according to any one of claims 1 to 29, comprising a light chain variable region VL, wherein the VL comprises a framework region L-FR1, the C-terminus of the L-FR1 is directly or indirectly linked to the N-terminus of the LCDR1, and the L-FR1 comprises an amino acid sequence as set forth in SEQ ID NO: 55.

31. The isolated antigen-binding protein according to claim 30, wherein the L-FR1 comprises an amino acid sequence as set forth in SEQ ID NO: 26 or 27.

32. The isolated antigen-binding protein according to any one of claims 30 to 31, wherein the VL comprises a framework region L-FR2, the L-FR2 is located between the LCDR1 and the LCDR2, and the L-FR2 comprises an amino acid sequence as set forth in SEQ ID NO: 56.

33. The isolated antigen-binding protein according to claim 32, wherein the L-FR2 comprises an amino acid sequence as set forth in SEQ ID NO: 28 or 29.

34. The isolated antigen-binding protein according to any one of claims 30 to 33, wherein the VL comprises a framework region L-FR3, the L-FR3 is located between the LCDR2 and the LCDR3, and the L-FR3 comprises an amino acid sequence as set forth in SEQ ID NO: 57.

35. The isolated antigen-binding protein according to claim 34, wherein the L-FR3 comprises an amino acid sequence as set forth in any one of SEQ ID NO: 30 to 33.

36. The isolated antigen-binding protein according to any one of claims 30 to 34, wherein the VL comprises a framework region L-FR4, the N-terminus of the L-FR4 is linked to the C-terminus of the LCDR3, and the L-FR4 comprises an amino acid sequence as set forth in SEQ ID NO: 58.

37. The isolated antigen-binding protein according to claim 36, wherein the L-FR4 comprises an amino acid sequence as set forth in SEQ ID NO: 34 or 35.

38. The isolated antigen-binding protein of any one of claims 1 to 37, comprising L-FR1, L-FR2, L-FR3 and L-FR4, wherein the L-FR1 comprises an amino acid sequence as set forth in SEQ ID NO: 26 or 27, the L-FR2 comprises an amino acid sequence as set forth in SEQ ID NO: 28 or 29, the L-FR3 comprises an amino acid sequence as set forth in any one of SEQ ID NO: 30 to 33, and the L-FR4 comprises an amino acid sequence as set forth in SEQ ID NO: 34 or 35.

39. The isolated antigen-binding protein according to any one of claims 1 to 38, comprising L-FR1, L-FR2, L-FR3 and L-FR4, and the L-FR1, L-FR2, L-FR3 and L-FR4 are selected from any one of groups of amino acid sequences as follows:
(1) L-FR1: SEQ ID NO: 26, L-FR2: SEQ ID NO: 28, L-FR3: SEQ ID NO: 30 and L-FR4: SEQ ID NO: 34;
(2) L-FR1: SEQ ID NO: 27, L-FR2: SEQ ID NO: 29, L-FR3: SEQ ID NO: 31 and L-FR4: SEQ ID NO: 35;
(3) L-FR1: SEQ ID NO: 27, L-FR2: SEQ ID NO: 29, L-FR3: SEQ ID NO: 32 and L-FR4: SEQ ID NO: 35; and
(4) L-FR1: SEQ ID NO: 27, L-FR2: SEQ ID NO: 29, L-FR3: SEQ ID NO: 33 and L-FR4: SEQ ID NO: 35.

40. The isolated antigen-binding protein according to any one of claims 1 to 39, wherein the isolated antigen-binding protein comprises a light chain variable region VL, and the VL comprises an amino acid sequence as set forth in SEQ ID NO: 59.

41. The isolated antigen-binding protein according to any one of claims 1 to 40, wherein the VL comprises an amino acid sequence as set forth in any one of SEQ ID NO: 36 to 39.

42. The isolated antigen-binding protein according to any one of claims 1 to 41, wherein the isolated antigen-binding protein comprises an antibody light chain constant region, and the antibody light chain constant region comprises an amino acid sequence as set forth in SEQ ID NO: 40.

43. The isolated antigen-binding protein according to any one of claims 1 to 42, wherein the isolated antigen-binding protein comprises an antibody light chain LC, and the LC comprises an amino acid sequence as set forth in any one of SEQ ID NO: 47 to 49.

44. A polypeptide comprising the isolated antigen-binding protein of any one of claims 1 to 43.

45. An immunoconjugate comprising the isolated antigen-binding protein of any one of claims 1 to 43 or the polypeptide of claim 44.

46. An isolated nucleic acid molecule(s) encoding the isolated antigen-binding protein of any one of claims 1 to 43.

47. A vector comprising the nucleic acid molecule according to claim 46.

48. A cell comprising the nucleic acid molecule according to claim 46 or the vector according to claim 47.

49. A method for preparing the isolated antigen-binding protein of any one of claims 1 to 43, comprising culturing the cell according to claim 48 under conditions that the isolated antigen-binding protein of any one of claims 1 to 43 can be expressed.

50. A pharmaceutical composition comprising the isolated antigen-binding protein of any one of claims 1 to 43, the nucleic acid molecule of claim 46, the vector of claim 47 and/or the cell of claim 48, and optionally a pharmaceutically acceptable carrier.

51. Use of the isolated antigen-binding protein of any one of claims 1 to 43, the polypeptide of claim 44, the immunoconjugate of claim 45, the nucleic acid molecule of claim 46, the vector of claim 47, the cell of claim 48 and/or the pharmaceutical composition of claim 50, wherein the medicament for preventing, alleviating and/or treating a tumor.

52. The use according to claim 51, wherein the tumor includes a tumor with a high expression of PD-1 or PD-L1.

53. The use according to any one of claims 51 to 52, wherein the tumor includes a solid tumor.

54. The use according to any one of claims 51 to 53, wherein the tumor includes breast cancer, lung cancer, gastric cancer and urothelial carcinoma.

55. A method for preventing, alleviating or treating a tumor, comprising administering the isolated antigen-binding protein of any one of claims 1 to 43 to a subject in need.

56. The method according to claim 55, wherein the tumor includes a tumor with a high expression of PD-1 or PD-L1, the polypeptide of claim 44, the immunoconjugate of claim 45, the nucleic acid molecule of claim 46, the vector of claim 47, the cell of claim 48 and/or the pharmaceutical composition of claim 50.

57. The method according to any one of claims 55 to 56, wherein the tumor includes a solid tumor.

58. The method according to any one of claims 55 to 57, wherein the tumor includes breast cancer, lung cancer, gastric cancer and urothelial carcinoma.

59. The isolated antigen-binding protein of any one of claims 1 to 43, for use in preventing, alleviating or treating a tumor.

60. The isolated antigen-binding protein of claim 59, the polypeptide of claim 44, the immunoconjugate of claim 45, the nucleic acid molecule of claim 46, the vector of claim 47, the cell of claim 48 and/or the pharmaceutical composition of claim 50, wherein the tumor includes a tumor with a high expression of PD-1 or PD-L1.

61. The isolated antigen-binding protein according to any one of claims 59 to 60, wherein the tumor includes a solid tumor.

62. The isolated antigen-binding protein according to any one of claims 59 to 61, wherein the tumor includes breast cancer, lung cancer, gastric cancer and urothelial carcinoma.

63. A method for inhibiting the binding of PD-1 protein to PD-L1 protein, comprising administering the isolated antigen-binding protein of any one of claims 1 to 43, the polypeptide of claim 44, the immunoconjugate of claim 45, the nucleic acid molecule of claim 46, the vector of claim 47, the cell of claim 48 and/or the pharmaceutical composition of claim 50.

64. A method of stimulating the secretion of cytokines in immune cells, comprising administering the isolated antigen-binding protein of any one of claims 1 to 43, the polypeptide of claim 44, the immunoconjugate of claim 45, the nucleic acid molecule of claim 46, the vector of claim 47, the cell of claim 48 and/or the pharmaceutical composition of claim 50.

65. A method for detecting the presence and/or content of PD-L1 protein, comprising administering the isolated antigen-binding protein of any one of claims 1 to 43 and/or the polypeptide of claim 44.

66. A kit comprising the isolated antigen-binding protein of any one of claims 1 to 43, the polypeptide of claim 44, the immunoconjugate of claim 45, the nucleic acid molecule of claim 46, the vector of claim 47, the cell of claim 48 and/or the pharmaceutical composition of claim 50.
